# NEW EUROPEAN PATENT SPECIFICATION

(11) **EP 0 800 804 B2**
(45) Date of publication and mention of the opposition decision: **26.10.2011**
(45) Mention of the grant of the patent: 25.06.2003
(21) Application number: 97201073.0
(22) Date of filing: 10.04.1997
(51) Int. Cl.: A61F 5/445

(54) **Fabrication of customized ostomy devices**
Herstellen von kundenspezifischen Ostomievorrichtungen
Fabrication d'appareils d'ostomie personnalisés

(30) Priority: 10.04.1996 US 631767
(43) Date of publication of application: 15.10.1997
(60) Divisional application: 02022497.8
(73) Proprietor: Bristol-Myers Squibb Company, New York, NY 10154 (US)
(72) Inventor: Cline, John B., New Brunswick, NJ (US); Konicky, Philip J., Newtown, Pennsylvania, PA (US); Johnsen, Kenneth A., Picataway, NJ (US); Kuczynski, Thomas J., Sayreville, NJ (US)
(74) Representative: Mays, Julie

(56) References cited:
- EP-A- 0 574 098
- WO-A-92/18074
- DE-A- 4 015 186
- DE-U- 9 105 753
- GB-A- 2 188 085
- US-A- 4 575 805
- US-A- 4 611 288
- US-A- 4 911 051
- US-A- 5 074 852
- US-A- 5 206 804
- US-A- 5 320 462

## Description

The present invention relates to a method for fabricating ostomy devices customized to the stomal topography and preferences of the user.

Certain surgical procedures known as colostomy, ileostomy and urostomy result in an opening in the abdominal wall, called a stoma, which permits waste discharge from the interior of a body cavity. Since the patient has no control over the waste discharge, it is often necessary for the patients who have undergone these surgical procedures to utilize an ostomy device to protect the stoma and collect the waste material as it is discharged.

Over the years, ostomy devices of a variety of different types and constructions have been utilized. Various materials and adhesives have been developed to increase the utility and wearability of the devices.

The basic device includes a collection receptacle or pouch connected to an adhesive coated wafer (also called a faceplate or label) which serves to mount the pouch to the body proximate the stoma. The pouch includes first and second thin barrier film walls which are sealed by heat welding or the like along the periphery. The pouch has an inlet opening defined by a plastic attachment ring which is designed to align with the stoma and an outlet for emptying material from the pouch. In single piece devices, the attachment ring of the pouch is permanently affixed to a corresponding plastic attachment ring affixed to the adhesive coated wafer. In two piece devices, the pouch is detachably mounted to the wafer by employing detachable rings, permitting the pouch to be replaced without removal of the wafer.

Wafers and pouches are manufactured in a variety of different standard shapes, sizes and configurations to meet the many different needs of the users. While these standard products meet the needs of the average user, they do not ideally meet the needs of any particular individual.

In most cases, the user must modify the product prior to use to suit his or her anatomy or lifestyle. Typical modifications which can be performed include cutting of the stoma receiving opening in the wafer, trimming the outside of the wafer, addition of convex inserts, application of paste and filters and folding or trimming the pouch.

However, even these modifications may not result in an ideal product for the particular individual. Users may have difficulty in performing the modifications, as well. This may be due to poor dexterity, poor eyesight or diminished mental capacity. Some desired modifications may be very difficult or impossible without special equipment. Some users may see making such modifications as taking too much time or as an undesirable task.

Thus, because of difficulties or simple reluctance on the part of the user, the modifications to the products are done poorly. As a result, product performance may suffer.

DE-A-4 015 186 discloses a procedure for customising the contact opening of an ostomy-care device by making a molding of the stoma contour and adapting the contour opening in accordance with this. EP-A-0 574 098 discloses a procedure for making an abstract or two-dimensional record of the contour of the stoma by using a scanning apparatus.

It is the general object of this invention to eliminate the above problems by fabricating ostomy devices which are customized so as to be uniquely suited to the needs and preferences of each individual user. This object may be achieved in a variety of different ways, using technologies of different sophistication and cost.

As the first step, the topography of the stomal area of the patient is carefully measured. This can be done manually, as the user is examined by an Enterostomal Therapist, photographically or by casting. It can be done by computerized touch probe, magnetic image resonance imaging (MRI), computerized axial tomography (CAT scan) or by laser scanning.

The resulting information is then recorded. Measurements taken by a therapist or other professional can be stored by marking a card or forming a template by a tracing cr cutout of the stoma opening and wafer outline. Information in this form is later converted into a digital image. Photos taken at different angles can be digitized to form an electronic image of the anatomy. Computerized touch probe, MRI and CAT scan equipment provide similar electronic size and shape maps. A laser scanner can be employed to provide 3-dimensional shape information either of the body itself or of a cast made of the body.

The digital information is placed in a database file. When an order is received, the information is retrieved from the file and used to determine of physical attributes of the customized wafer and pouch. For example, the size, shape and location of the stoma receiving opening in the wafer, the wafer outline, adhesive type and surface contour can be selected. The pouch size, shape, tail location, tap type, tap location, flange style and filter location can all be choosen.

Once the physical attributes are determined, the appropriate tooling is selected and adjusted by the computerized controller for the fabrication equipment. Cutting of the wafer hole and outline is done with the cutting tool mounted onto an X-Y table. The cutting tool itself could be a laser, a high pressure water jet, a hot wire, a high speed grinding/machine tool, a knife, a saw, a RF scalpel or be accomplished by nibbling or punching. The desired surface contour of the wafer may be achieved by vacuum forming barrier adhesive over filler material molded into the required shape, over a base formed by multiple slices joined together to approximate the surface contour or over a planar base upon which protrusions of various shapes are affixed and into which valleys are formed. Convex disks and inserts can be used and if necessary supplemented with shape altering material.

Pouches are customized by selecting the outline configuration and welding the periphery by a laser, ultrasonic horn, hot air weld nozzle or hot roller mounted on an X-Y table. Filters and taps can be welded at desired locations.

After the order is completed, the products may be labeled with the name or code number of the customer. The products are then packed and shipped to the customer in accordance with instructions from the customer. The topographical, preference information and patient history are kept in the electronic file for future applications. Stoma site examination is only required once, except that stoma site changes may require additional examination.

It is therefore another object of the present invention to provide a method for fabrication of customized ostomy devices wherein the topography of the stoma area is measured, the information relating to the measurement of the stomal area is recorded, transformed into electronic form and stored in a database file along with patient history and preference information.

It is another object of the present invention to provide a method for fabrication of customized ostomy devices wherein the electronic measurement information is utilized to determine the physical attributes of the customized ostomy device.

It is still another object of the present invention to provide a method for fabrication of customized ostomy devices wherein ostomy devices are automatically fabricated to have customized physical attributes by computerized control equipment.

Accordingly, the invention provides the method of claim 1.

In accordance with the present invention, a method is provided for fabricating customized ostomy devices including an adhesive wafer and a collection pouch as described in claim 1. The topography of the stomal area of the patient is measured. Information relating to the measurements of the stomal area is recorded. The recorded information is transfered into electronic form and stored in a database file. The electronic information is utilized to determine the physical attributes of the ostomy device. The ostomy device is then fabricated to have the customized physical attributes determined in accordance with the record information.

The recorded information is utilized to select the size, shape and location of the stoma receiving opening in the adhesive wafer. The adhesive wafer is fabricated to have a stoma receiving opening of the selected size, shape and location.

The information is utilized to select the size and shape of the adhesive wafer. The outline of adhesive wafer is fabricated in accordance with the selected size and shape.

The information is utilized to select the surface contour of the adhesive wafer. The adhesive wafer is fabricated with the selected surface contour.

The information is utilized to select the size and shape of the pouch. The pouch is formed having the selected size and shape.

The information is utilized to select the location of the attachment ring in the pouch wall. The pouch is fabricated with the attachment ring positioned at the selected location.

The information is utilized to select the location and type of tap. The pouch is fabricated with the tap at the selected location and of the selected type.

As part of the patient examination, information as to patient history and preferences is obtained and recorded. This recorded information is used to select the type of adhesive to be used in the wafer. Adhesive of the selected type is formed on the backing to fabricate the adhesive wafer.

This recorded information is also used to select a barrier film or the pouch wall. The pouch is fabricated with walls composed of the selected barrier film.

The recorded information is also used to select a barrier film color. The pouch walls are fabricated of a barrier film with the selected color.

The recorded information is used to select the appropriate accessories to be supplied with the ostomy device. Such accessories may include convex inserts, tail clips and the like.

The recorded information is utilized to select whether or not the pouch is to be detachably coupled to the adhesive wafer. The ostomy device is fabricated with means for detachably coupling the pouch to the wafer, if a two piece appliance is selected.

The step of measuring the topography of the stomal area may include manual measurements, in which case a card or template may be the recording medium. Photographs, CAT scanning, MRI, computerized touch probing or laser scanning of the stomal area may be employed to form an electronic image of the scanned area.

Alternatively, a mold can be formed of the stomal area and thereafter scanned by the laser. The mold can be formed by filling a collar with mold material. The filled collar is then placed adjacent the stomal area and the material allowed to solidify.

The outline shape of the adhesive wafer may be customized by starting with a wafer having a size which is larger than the selected size. The wafer is then cut to have an outline in accordance with the selected size and shape. This can be done by a cutting tool, such as a laser, water jet, knife or the like mounted on a computer controlled X-Y table.

A wafer with the selected surface contour may be formed by employing a convex disk with a central opening. The convex disk is affixed on the wafer. The surface of the convex disk is coated with the adhesive.

A convex insert may be employed. The insert is snap fitted into the central opening of the convex disk.

An annular enclosure defining an internal chamber may positioned over the convex disk surface, prior to coating same with adhesive. A contour altering material is introduced into the chamber. The material is manipulated to alter the contour of the wafer. The material may comprise corn starch, a liquid or a quick curing resin.

The pouch may be formed by placing sheets of barrier film one on top of the other. The surface of one sheet is welded to the surface of the other sheet as the welding instrument is guided along an outline determined in accordance with the recorded information, to form the pouch periphery. The welding instrument may be a hot roller, ultasonic horn, hot air welder or nozzle mounted on a computer controlled X-Y table.

The welding instrument may comprise a laser beam. The beam may be guided by moving a mirror to reflect the beam onto the sheets.

An attachment ring is affixed to the wafer. A customized flange may be received on the attachment ring. A first coupling ring may be affixed to the attachment ring. A second coupling ring may be affixed to the pouch.

The step of recording the measurement information includes the step of marking a card to indicate various aspects of the topography of the stomal area. The step of transforming the recorded information into electronic form includes the step of scanning the card surface and converting the scanned information into digital form.

The step of recording the measurement information includes the step of forming simulated parts of the wafer, altering the simulated parts of the wafer in accordance with the measurements and assembling the altered parts in a manner indicative of the desired relationship between the parts. The step of transforming the recorded information includes the step of scanning the simulated parts and converting the scanned information into digital form.

An ostomy device customized for a particular user is provided including an adhesive wafer with a stoma receiving opening. An attachment ring is mounted on the wafer and defines the area in which opening is situated. A waste collection pouch is provided having an inlet. Means are provided for mounting the pouch on the wafer with the inlet aligned with the opening. The size, shape and location of the stoma receiving opening within the defined area is selected in accordance with the size, shape and location of the stoma of the user.

The wafer has a shape and size selected in accordance with the topography of the stomal area of the user.

The pouch has a contour selected in accordance with the topography of the stomal area of the user. The pouch includes an attachment ring. The location of the attachment ring on the pouch is selected in accordance with the topography of the stomal area of the user.

The surface contour of the wafer is selected in accordance with the topography of the stomal area of the user.

A customized ostomy device is provided including an adhesive wafer with a stoma receiving opening and a waste collection pouch with an inlet. Means are provided for attaching the wafer and the pouch with the opening aligned with the inlet. The attaching means includes a first attachment ring welded to the pouch, a flange removably mounted on the first ring and a second attachment ring welded to the wafer. Either the first ring or the flange includes an annular channel. The second ring includes an annular protrusion adapted to be removeably received within the channel.

The first ring has an exterior wall. Protrusions are provided on the wall spaced to receive the flange therebetween. The flange is formed of material flexible enough to pass over the protrusions in one direction.

Means are provided for removeably mounting the flange on the first ring. The removeable mounting means include a recess in the first ring and a protrusion on the flange adapted to be received within the recess.

A customized ostomy device is provided including an adhesive wafer, a waste collection pouch and means for mounting the pouch on the wafer. The pouch includes a wall. A tap mounting washer is affixed to the pouch wall over an opening in the wall. The washer includes a central opening aligned with the pouch opening and a second opening offset from the central opening. A removeable disk is provided in the second opening. A tap is provided including a base with a central post adapted to be received within the central opening in the washer.

The post may be hollow and act to connect the tap to the interior of the pouch. The post may be solid and function as an axis about which the base may be rotated relative to the washer.

The tap includes a conduit. The conduit terminates in the base so as to align with the second opening when the base is in a selected rotational position relative to the washer.

In with these objects and those which may hereinafter appear, the present invention relates to fabrication of customized ostomy devices as set forth in detail in the following specification and recited in the annexed claims, taken together with the accompanying drawings, wherein like numbers relates to like parts and in which:
Fig. 1 is a schematic drawing of the overall method of the present invention;
Fig. 2 is a schematic drawing illustrating typical one piece and two piece customized ostomy products which can be obtained using the method;
Fig. 3 is a schematic diagram illustrating the features of the ostomy device which can be customized;
Fig. 4 is a plan view of a typical card upon which measurement information can be recorded;
Fig. 5 illustrates three parts used to simulate the customized wafer, prior to cutting;
Fig. 6 is an exploded view of the parts illustrated in Fig. 5, after cutting;
Fig. 7 is an isometric view of the assembled parts of Fig. 6;
Fig. 8 illustrates the steps in making a mold of the peristomal area;
Fig. 9 is an isometric view of a wafer with a customized stoma receiving opening;
Fig. 10 is a plan view of a wafer with a customized contour;
Fig. 11 is an isometric view of a wafer with a customized surface contour;
Fig. 12 is a plan view of a wafer mold built of multiple slices;
Fig. 13 illustrates the slices which make up the mold of Fig. 12;
Fig. 14 is a cross-sectional view of a wafer with a conventional convex disk;
Fig. 15 is a wafer with a surface contour formed by injecting shaping material;
Fig. 16 is a cross-sectional view of a wafer with an annular pouch filled with corn starch;
Fig. 17 is a cross-sectional view of a wafer with an annular pouch as it is filled with a shaping liquid;
Fig. 18 is a partial cross-sectional view of a wafer with a compressed sponge material in the pouch;
Fig. 19 is a view similar to Fig. 18 with the sponge expanded;
Fig. 20 is a partial cross-sectional view of the wafer with the injection needle in place;
Fig. 21 is a partial cross-sectional view of the wafer with the valve closed;
Fig. 22 is a partial cross-sectional view of a wafer with an integral chamber before it is closed;
Fig. 23 is a partial cross-sectional view of the wafer of Fig. 22 with the chamber closed and filled;
Fig. 24 illustrates pouch sealing by laser;
Fig. 25 illustrates pouch sealing by hotwheel;
Fig. 26 is a side elevational view of a part welder;
Fig. 27 is a plan view of the film showing three different pouch configurations;
Fig. 28 is an elevational view of a portion of a pouch with minimal head room;
Fig. 29 is an exploded view of a one piece device;
Fig. 30 is an exploded view of a pouch with a flange having a channel adapted to receive a detachable mating ring;
Fig. 31 is a plan view of a pouch with an attachment ring and flange;
Fig. 32 is an exploded view of a ring with a channel and a snap-fit type flange;
Fig. 33 illustrates one version of the snap-fit structure;
Fig. 34 illustrates a second verison of the snap-fit structure;
Fig. 35-40 show different structures of a two part attachment ring;
Fig. 41 shows three different tap types and a washer for mounting same;
Fig. 42 is a cross-sectional view of a nonrotary tap mounted on the washer;
Fig. 43 is a cross-sectional view of a rotary tap type mounted on the washer;
Fig. 44 illustrates a first preferred embodiment of a tail clip;
Fig. 45 illustrates the custom cutting of the clip of Fig. 44;
Fig. 46 illustrates a second preferred embodiment of a tail clip; and
Fig. 47 illustrates the custom cutting of the clip of Fig. 46.

In general, the present invention presents a radically different approach to ostomy device design and fabrication in which the object is to produce appliances which are customized to the anatomy and preferences of the user. Thus, instead of producing wafers and pouches in a variety of standard sizes and shapes and attempting to make minor modifications in the field, aimed at meeting the needs of theoretical "average" patient, the ostomy devices produced according to the present invention will be custom designed and fabricated to suit the needs of the individual. No modification will be necessary in the field and optimum results and comfort are achievable.

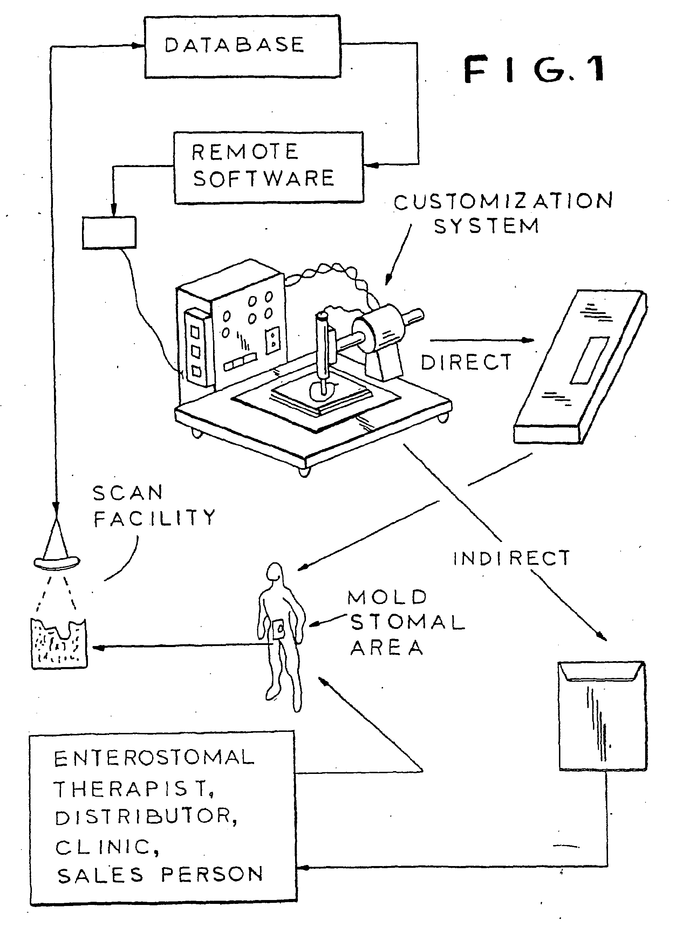

The overall method is illustrated schematically in Figure 1. It begins with an examination of the patient to obtain measurements of the stomal area. The measurement information is then recorded. In the figure, this is represented by taking a mold of the stomal area. This can be done by the Enterostomal Therapist, a salesperson with the appropriate skill at a clinic or the like. However, as described below, may other methods of measurement and recordation are possible.

The mold is taken to a facility where it is scanned, such by a laser scanner. The scanner forms a digital image or map of the contours of the mold material, which of course represents the anatomy of the patient. The digital image is stored in an electronic patient file in a database.

The digital image and other patient information, such as patient history and preferences are kept in the file. The appropriate software and the file information are loaded into a computerized controller, which determines the physical attributes of the wafer and pouch in accordance with the recorded information. The controller selects and adjusts the appropriate tools tc perform the fabrication work and guides the machines, such as the computerized laser cutter illustrated, to perform the fabrication operations. The laser cutter is illustrated cutting the stoma receiving opening in a wafer.

Once fabrication of the wafers and pouches are complete, they are packaged and shipped either directly to the customer, or to the therapist, salesperson or clinic responsible for examining the patient and distribution to the patient.

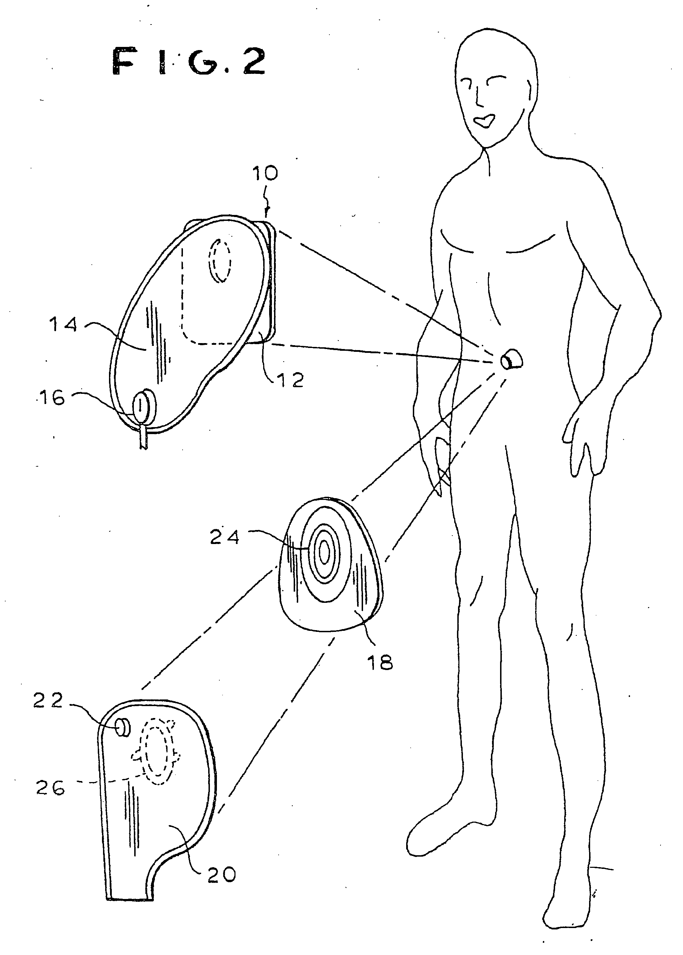

Figure 2 illustrates a patient with a stoma. Two different versions of the customized ostomy device, a one piece device and a two piece device, are shown. The one piece ostomy device 10 consists of a generally rectangular adhesive wafer 12 and a pouch 14 connected thereto. Pouch 14 has a customized contour and a rotatable drain tap 16. A two piece applicance is also shown. The two piece appliance comprises a wafer 18 with a customized contour and a pouch 20 with a filter 22. Wafer 18 has a coupling ring 24 which is adapted to detachably mount to a mating coupling ring 26 affixed to pouch 20.

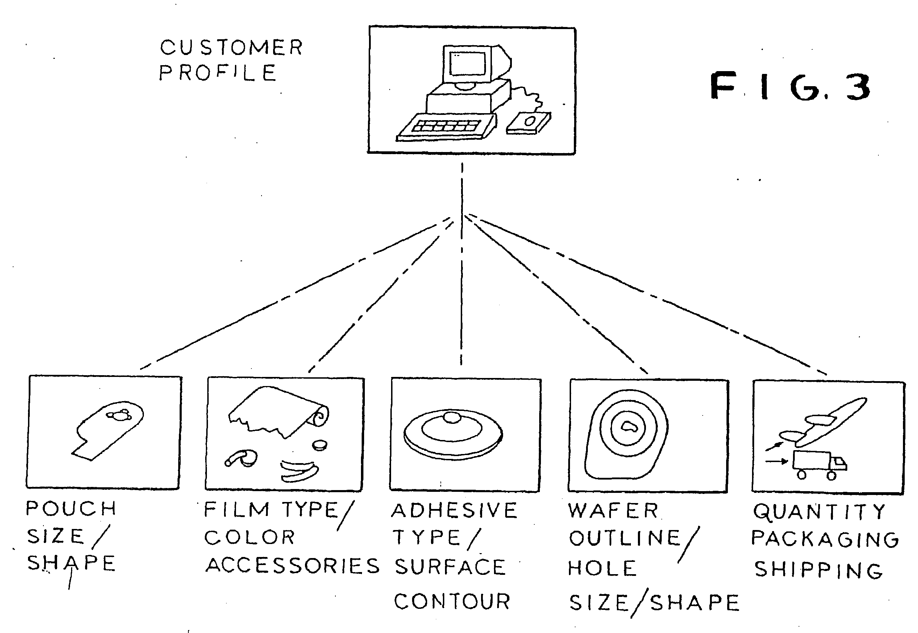

Figure 3 schematically illustrates the types of design features which could be customized. The customer profile is stored in a computer database. The profile may include a digital image of the patient's anatomy, the patient's history, including any allegeries to particular adhesives or films and preferences for adhesives, color, type of appliance (one piece or two piece), quantity for each order, packaging and shipping methods.

With this information on file, the computer can determine the physical attributes of the customized wafer and pouch. The pouch size and shape can be selected, along with the film type, color and required accessories. The type and contour of the wafer adhesive can be determined. The shape, size and location of the stoma receiving hole in the wafer and the wafer outline can be chosen.

Moreover, the quantity required can be calculated based on past order frequency. How the devices are to be packed and the method of shipping are also determined.

Once these decisions are made, the information is used with the appropriate software to select the appropriate materials, fabrication tools and to guide the fabrication tools. It is used to determine the quantities to be made, how many are to packed and how they are to be shipped.

### MEASUREMENT OF THE STOMAL AREA

Regardless of the fabrication method, the first step is an examination of the patient and careful measurement of the contour of the stomal area to develop a digitized 2-dimensional or 3-dimensional image or map of the topography of the anatomy of the patient. Measurement, recordation and digitization of the measurements can be done in a variety of different ways.

One simple method is to measure the stomal area manually and record the measurements on a card. The card allows the ostomate or professional to convey measurement information to an automated customized manufacturing system in an efficient and convenient manner. The recorded information may include the attributes of the size and shape of the stoma receiving hole in the adhesive wafer, along with the wafer outline size and shape, the relation between them and the relation of same with other components. The card may also display the limits of the system for particular products and sizes, and the optimum size or product which is best suited for the application.

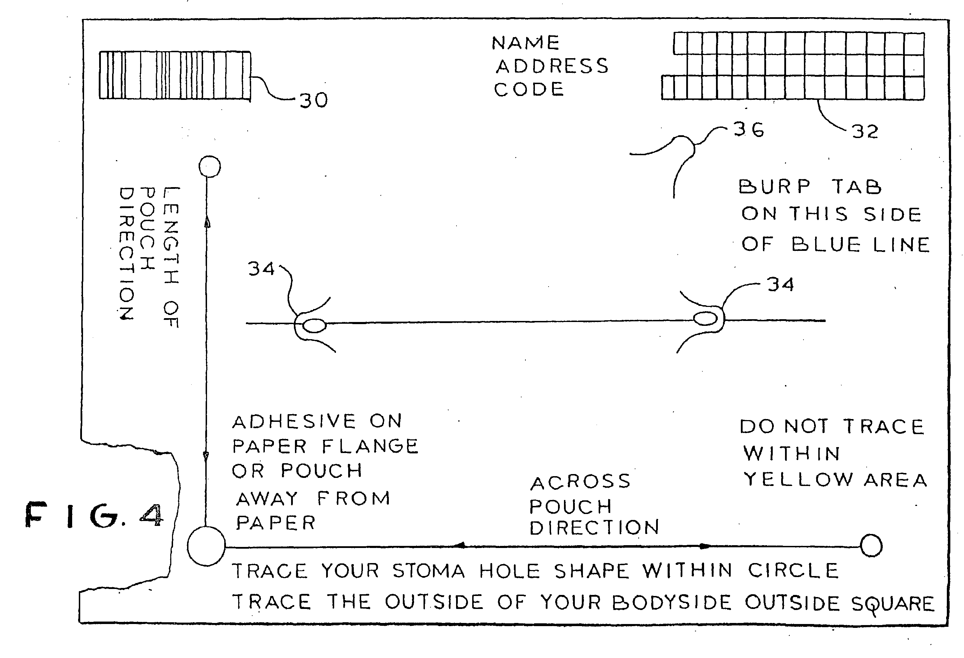

Some of the features of a typical card are illustrated in Figure 4. The card generally designated 28 may be composed of paper, foil, plastic, film or composites of same which can be subjected to mailing, shipping or transmitting without damage or loss of accuracy. Detachable copies of the card may be made to permit the user or professional to retain a record of the information.

The information on the card may be formed by drawing or tracing the stoma size, shape and location and the relationships between the stoma and other physical attributes of the anatomy. This may be done with a special pencil, pen or marker which enables the card to be automatically read. Another approach is to cut a hole can be cut into the card to indicate the size, shape and location of the stoma.

An adhesive wafer or a similarity thereof could be cut as desired and applied to the card. A special tape, foil or film could be cut and applied to the card. Even a piece of paper simulating a wafer could be cut and applied to the card. Components of the card could be assembled in a manner representing the desired size, shape and location of the components of the product.

Card 28 may carry a distinct bar code type label 30 which identifies the patient. The label is read by the system to automatically index the attributes in a computer file. The label is shown as being located in the upper left of the card.

An area 32 for the patient's name, address and other information may be provided. This area is read by the system, the information is stored and may be used to generate a shipping label Area 32 is shown as being situated in the upper right of the card.

Location holes or spots which are used by the system as datums for measuring size, shape and relationships of attributes may be provided on the card. The scheme of the holes orient and scale the information on the card for automatic processing by the system. These can be located near the bar code label in the lower left and lower right of the card.

Various symbols may be used on the card to guide the user to furnish attribute information accurately. These may include representations of product characteristics such as belt attachments on flange tabs 34; burp tabs 36, colored, shaded or outlined areas; colored lines or instructional notes. These may be located near the center or lower edge of the card.

Special inks which are detected or not detected by the system as part of the automated reading processes may be used to print the symbols. Any of the features may also be punched, cut, laminated, coated or embedded.

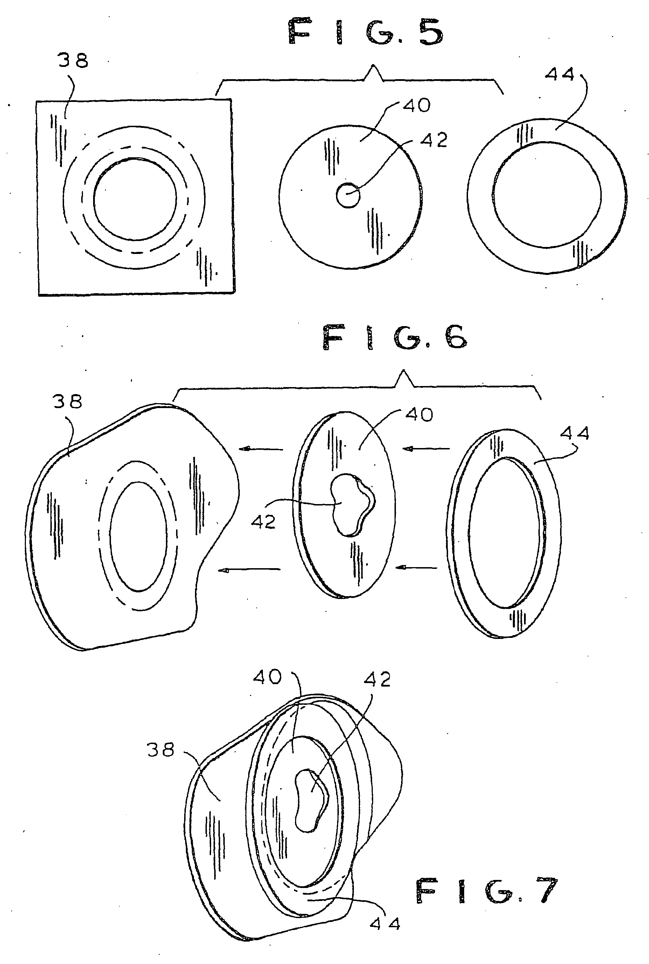

Another approach, which is illustrated in Figs. 5, 6 and 7 would be to provide a template of simulated components stamped from a sheet of a self-adhering plastic, such as vinyl. The user would remove each components, one at a time, from the sheet and cut or trim the simulated component to indicate the desired characteristics of the wafer.

The main component would be a clear square sheet 38 which could be trimmed to the exact size and shape desired by the end user as shown in Fig. 6. Another component would be a disk 40, made of the same material, which would feature a small central "starter hole" 42. This hole 42 could be trimmed to the exact size and shape required by the user to fit his/her stoma. Once trimmed, this component could be attached to the first component 38 in the location and orientation desired by the user. In a similar manner, a component 44 representing the attachment ring could be mounted on the first two components to represent the desired flange placement. In a similar manner, other features such as border material could also be simulated, trimmed to the desired shape and size, and located on the assembly as required by the user. Contrasting colors of each component would enable the size, shape, and relative location of all of the components to be scanned and recorded. Furthermore, the nature of the assembly and the materials used would allow the user to "trial fit" the assembly to ensure proper fit.

The information the card or template of simulated components must be read and converted into digital form for storage and use by the system. This could be done manually, by an operator observing the card or simulated components and entering data into a computer via a keyboard. A mechanized reading method could be used as could electronic, magnetic or light sensing techniques, or some combination thereof.

Somewhat more sophisticated measuring techniques are also available. One perferred method is to make a mold of the peristomal area and thereafter scan the mold to create a digital image.

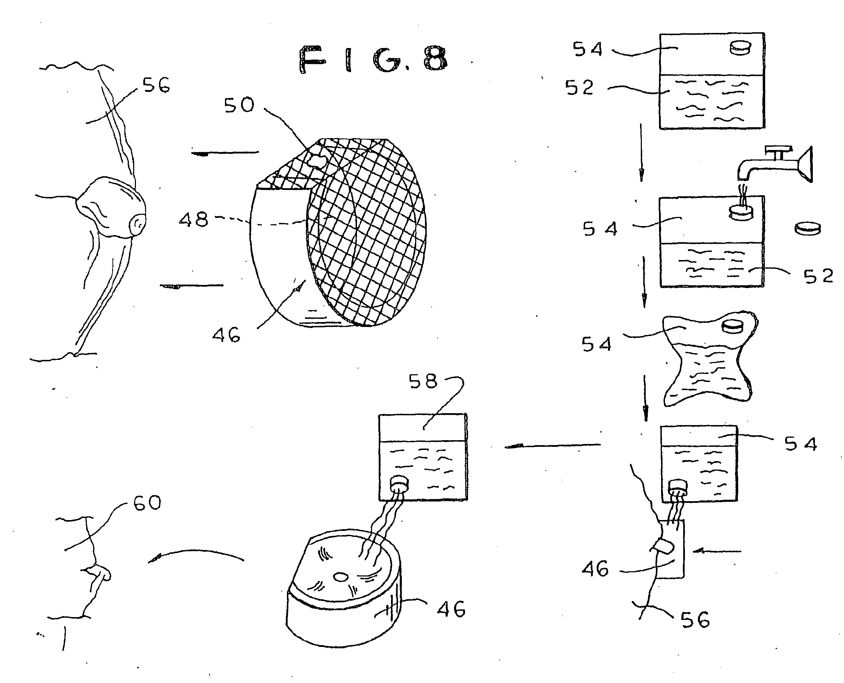

As illustrated in Fig. 8, a soft foam "horse collar" 46, composed of plastic mesh can be constructed with an open side 48 and an inlet 50 in the top surface. A pre-mixed aliginate 52 in a plastic bag 54 is provided. Water is introduced into the bag 54 and the material is kneaded to the required consistancy.

Holding form 46 against the body 56 with the stoma received in the open side 48, the mold material is poured in the opening 50 in the top of the form 46 and permited to harden. The mold 46 is then removed and pre-mixed plaster is poured in from a bag 58 and allowed to harden to form a plastic reproduction 60 of the stomal area. The plaster reproduction could be transported to another location where a laser scanner is employed to obtain a digital image. The plaster reproduction could even be used directly to create the product, if required.

Another measurement possibility is to use photographic images. Photographs of the stoma could be taken at different angles. Three dimensional information as to the size and shape of the stoma could thus be recorded. The photographies could be transferred to a facility to be digitized to form an electronic image.

Computerized touch probing with a pencil shaped touch probe interfaced with a personal computer could be employed. The probe touches many locations around the stoma. At each point, the location relative to the other touch points is recorded in the computer. The electronic size and shape map of the stoma site formed in this way can be transferred and used in the system.

Laser scanning can also be employed. Laser scanning of the stoma site (or a plaster reproduction) can provide an electric file with 3-dimensional size and shape information for use in the system.

### STORAGE OF THE MEASUREMENT INFORMATION

Depending upon how the measurements are taken, the information is read, if in card form, or scanned, if in the form of a simulation, a plaster cast or photographs and digitized. MRI, CAT scan, touch probe and laser scanning equipment may generate a digitized image directly.

The digitized measurement data is electronically transferred to a customer file database. In addition, other pertinent information such as customer preferences and history of product usage or allergies to certain materials is recorded into the database as well.

### UTILIZING THE DIGITIZED INFORMATION TO DETERMINE PHYSICAL ATTRIBUTES OF THE DEVICE

Once all of the information has been recorded and digitized, it is stored in a customer file database. Each time an order from a particular user is received, the user's file is retrieved and the data downloaded into a computer which has the appropriate software to select the physical attributes of the customized wafer and pouch to be fabricated.

Such features which can be customized include the size, shape and location of the stoma receiving opening in the wafer, the wafer outline, the adhesive type and surface contour. With regard to the pouch, the size, shape, tail location, tap type and location, style (two piece or one piece) and filter location can be choosen. In addition, accessories such as clamps or belts can be designated for inclusion in the packaging.

### FABRICATION OF THE OSTOMY DEVICE

Once the physical attributes of the ostomy device have been selected, this information is downloaded into a computerized equipment controller which selects and adjusts the required tooling. Thus, a tool for cutting the stoma receiving hole in the wafer and wafer outline is selected and mounted on an X-Y table to be guided through the cutting process. Likewise, a tool for welding the pouch periphery is mounted on an X-Y table. Equipment for affixing the attachment ring, tap and filter to the pouch wall is selected as well.

The controller directs each operation and fabricates the desired number of wafers and pouches of the selected materials. It then causes the wafers, pouches and accessories to be packed and shipped.

### CUSTOMIZATION OF THE ADHESIVE WAFER

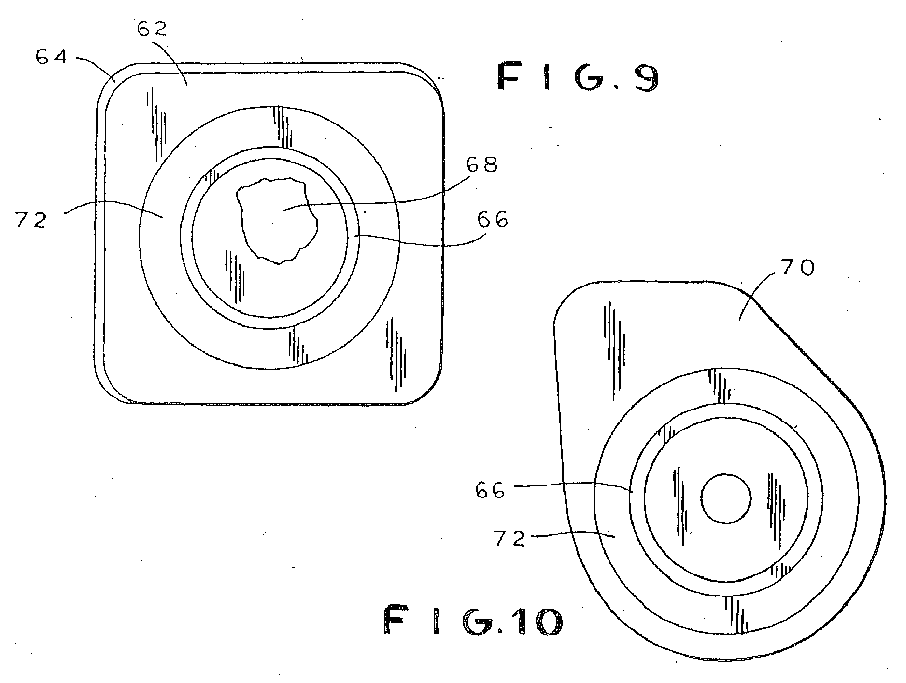

The wafer illustrated in Fig. 9 consists of a backing 62 which may be made of a non-woven polyethlyene material. A pressure sensitive adhesive layer 64 is cast onto the backing. The adhesive layer 64 is preferrably 0.1 mm (4 mils) thick and can be an acrylic microporous adhesive as taught by Copeland in U.S. Patent No. 3,121,021, a microporous hydrocolloid adhesive as taught by Cilento in U.S. Patent No. 4,427,727, or a polyisobutylene - hydrocolloid containing adhesive as taught by Chen in U.S. Patent No. 3,339,546, by Chen et al. in U.S. Patent No. 4,192,785, by Pawelchak in U.S. Patent No. 4,393,080, or it can be adhesive composition containing a styrene type block copolymer in addition to the polyisobutylene and hydrocolloids as taught by Doyle et al. in U.S. Patent No. 4,551,490. The particular adhesive selected used may depend upon medical necessity, such as allergies or upon patient preference.

On the non-adhesive side of film 62 a plastic attachment ring 66 is welded. This ring is used to affix the pouch to the wafer. This may be accomplished in a nondetachable manner, so as to form a one piece appliance or in a detachable manner, to create a two piece applicance, wherein the pouch may be removed for cleaning or replacement without removal of the wafer from the body.

The attachment ring 66 on the wafer defines the area within which the stoma receiving opening 68 is situated. As shown in Fig. 9, within the confines of the ring, the opening 68 can be of any size, shape and location.

Likewise, the outline 70 of the wafer can be cut to any configuration, such as the shape illustrated in Fig. 10. Of course, the wafer cannot be smaller than the base 72 of the attachment ring and there must be sufficient surface area for the adhesive to securely adhere.

The stoma receiving opening 68 and outline 70 are cut using a cutting tool mounted on an X-Y table controlled by the control computer. A variety of know technologies can be utilized for this purpose. A high speed grinding/machine tool (preferrably a cone shaped tool is employed to provide a beveled cut which will allow better skin protection near the stoma), a wafer jet cutter, a knife, a saw, a laser cutter, a hot wire or an RF scapel can be used. A nibbling or punching technique may also work well.

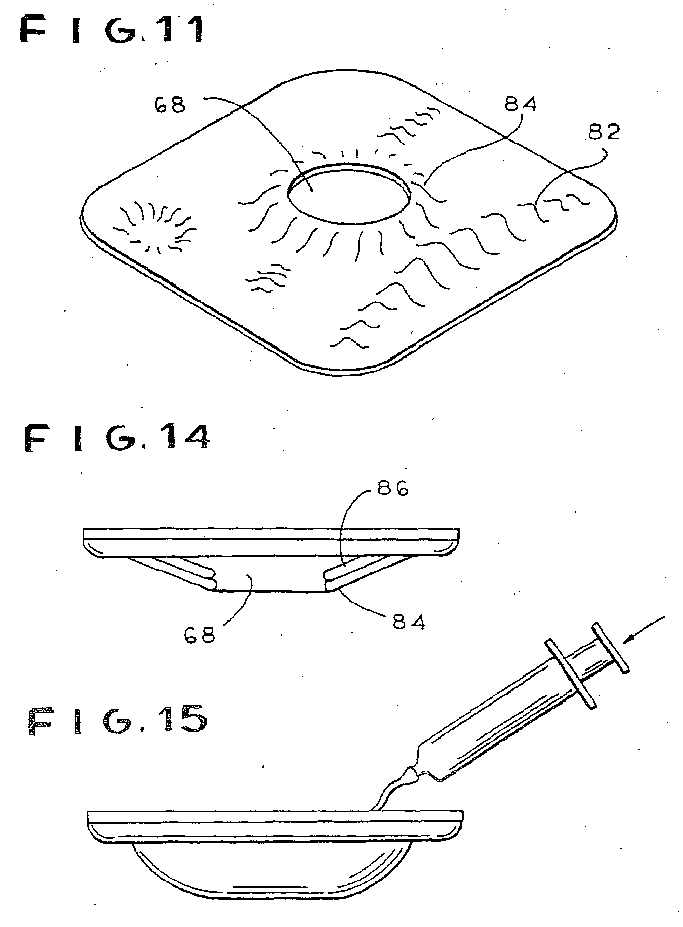

As illustrated in Figure 11, the surface contour of the wafer may be adjusted to conform with skin surface irregularities such as folds, crevices and depressions. One way to form a wafer with the desired surface contour is by heating and forming the barrier adhesive into a mold. The adhesive may be backed up by other materials which have the necessary filling properties, but are easier to handle and lower in cost than the barrier adhesive.

The filler material may be formed first to the desired shape, and then the barrier adhesive is vacuum formed over it. Methods other than heat may also be employed to form the shaped barrier adhesive, such as UV light curable materials.

The mold that is made to form wafers with a surface contour may be made employing several other techniques as well, including sterolithography, machining by computer aided manufacturing (CAD/CAM) process or by a sintered powdered metal technique.

Another method is to use a flexible array of pins. The array of pins forms the shaped mold by having pins project into the mold to a degree which is based on an electronic signal. This technique represents a truly flexible approach to tooling.

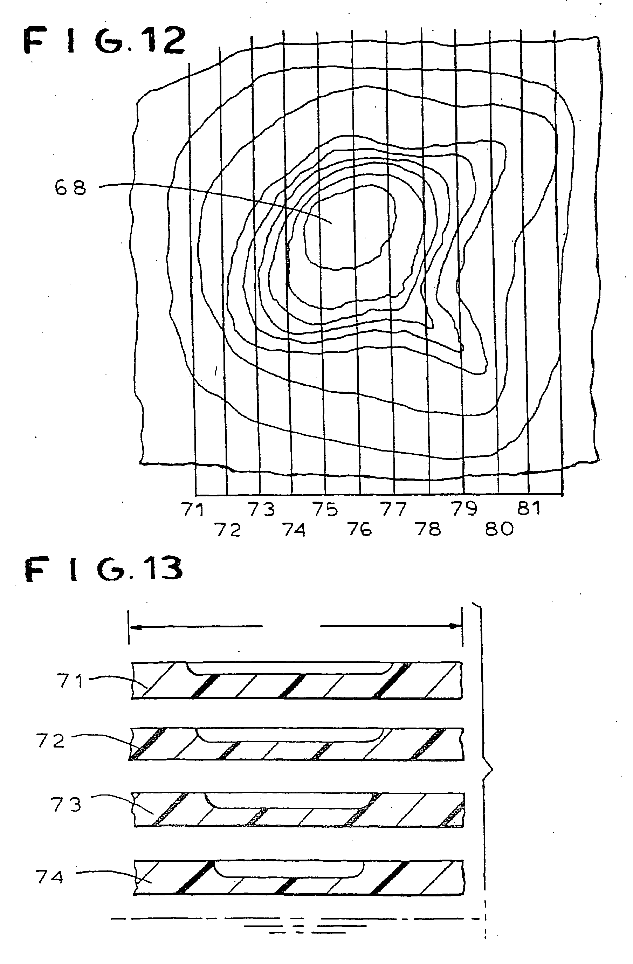

Another method of preparing the mold in accordance with the information in the database is illustrated in Figures 12 and 13. The mold is built by cutting and stacking a number of cross-sectional layers 71-81, one next to the other. Each layer cr slice 71-81 could be formed by cutting a thin sheet into the desired configuration by a water jet cutter or any other suitable instrument. Slices, such as illustrated in Fig 13 are then bonded together to form an approximation of the desired surface contour. The accuracy of the mold increases as the number of slices increases.

Another way to form protrusions 82 on the surface of a wafer is the use of customized parts or sections of particular shapes and sizes which are positioned and affixed to the surface of the film prior to coating with the adhesive. Such sections can stick on or snap into place on the film.

It has been previously recognized that providing the wafer with a convex surface 84 results in a substantially improved seal with the skin for stomas of particular topography. Accordingly, it is known to employ an annular rigid on semi-rigid plastic disk 86 with a convex surface as illustrated in Figure 14-between the wafer film and adhesive to provide a wafer with the desired convex surface contour.

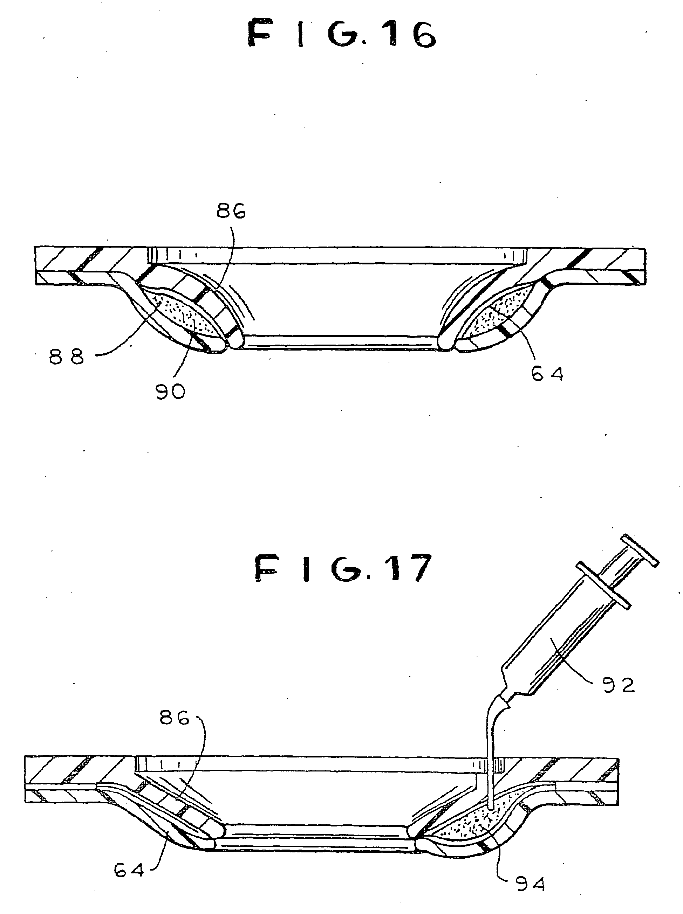

However, it is often desirable to provide the surface contour with a more customized convex shape than the simple conical shape obtained by the use of a conventional convex disk, as shown in Fig. 15. Accordingly, additional material, in the form of a powder or liquid, can be used to build up the convex surface to have the desired contour. This technique is illustrated in Figs. 16 to 23.

An annular shaped hollow pouch 88 is affixed by adhesive or the like to the convex surface of the disk 86. In Fig. 16, the pouch 88 chamber is filled with corn starch 90. The wafer is then coated with adhesive 64. The adhesive 64 can be manipulated to obtain the desired surface contour. The corn starch 90 will hold the shape.

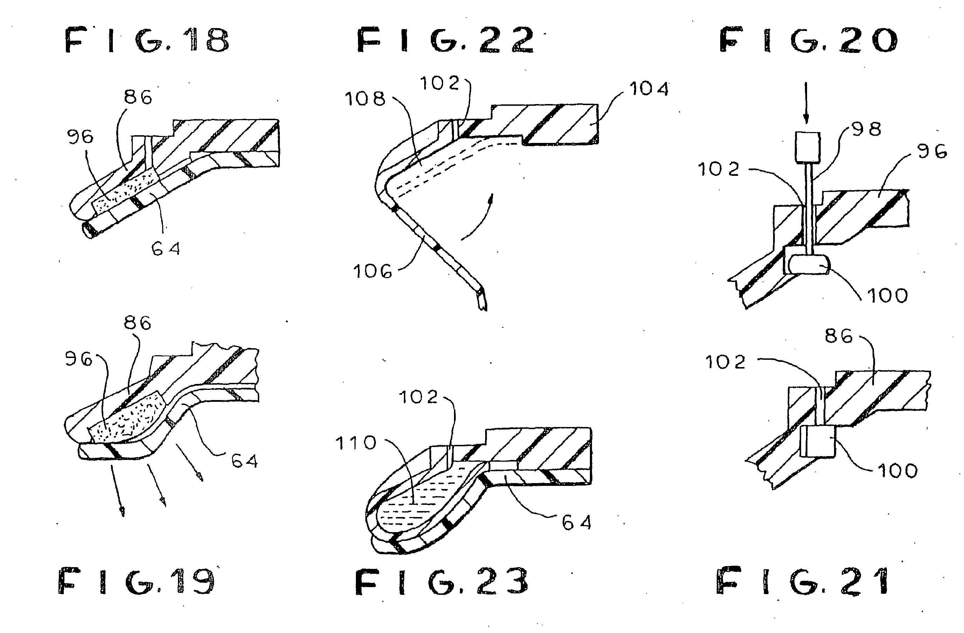

Alternately, as illustrated in Fig. 17, a syringe 92 can be used to inject water, oil or a quick setting resin 94 into the annular pouch cavity. In Fig. 18 a compressed sponge or "fizzies" 96 is situated within the cavity. Water is then injected into the cavity to expand the material to the desired extent, as illustrated in Fig. 19.

As seen in Figs. 20 and 21, the fluid can be injected through a hollow metal needle 98 of the type used to inflate footballs and basketballs. Embeded within the convex disk 86 is a compressible valve clyinder 100 which is compressed by the needle (Fig. 20) to allow fluid into the chamber, but seals the inlet opening 102 when the needle is removed (Fig. 21).

Figures 22 and 23 illustrate a method of forming an intergral chamber as part of the convex disk. As shown in Fig. 22, disk 104 is formed with a cover portion 106 which is bent and sealed into position (shown in phantom in Fig. 22) so as to form chamber 108. Thereafter, liquid 110 is injected through opening 102 to form the required curvature and adhesive 64 is deposited on the curved surface.

### CUSTOMIZATION OF THE COLLECTION POUCH

The pouch has a front barrier film wall and a rear barrier film wall. The walls are made of thin, flexible film which is heat welded around the periphery to form an enclosed receptacle. Depending upon the type of stomal discard, the pouch may include drainable outlet which is sealed with a clip or it may include a liquid drainable tap valve. Otherwise, the bottom may be sealed in the same manner as periphery.

The films from which the pouch walls may be made are selected from materials which possess the properties pf being moisture impermeable, odor impermeable and capable of being heat sealed or impulse welded. Suitable materials include polyethylene, copolymers of polyethylene and ethylene vinyl acetate, co-polymers of vinyl chloride and polyvinylidene chloride and laminates thereof. The pouch walls are preferably from about two to four mils thick.

Pouches may be customized by moving the tail location, changing the outline, changing the attachment ring location, changing the flange type, changing the filter and tap location or the tap type. These changes can be achieved in a number of different ways.

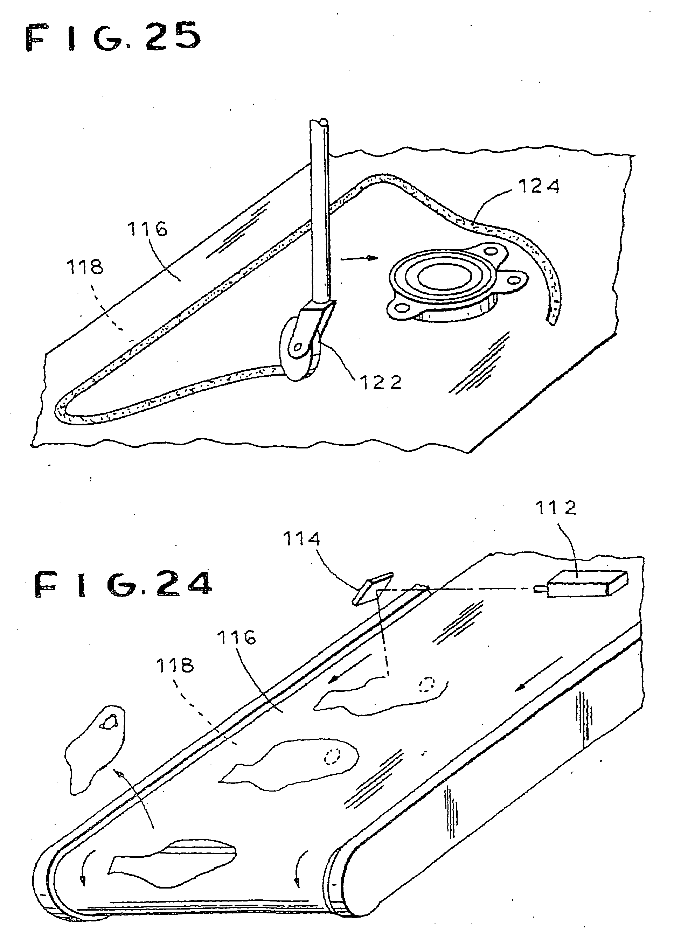

The outline may be welded by using a laser beam, as illustrated in Fig. 24 or a hot roller mounted on a X-Y table, as illustrated in Fig. 25. Alternately, the weld can be formed ultrasonically or by a hot air weld nozzle.

As shown in Fig. 24, the laser 112 generates a beam which is reflected by a mirror 114 onto two sheets of barrier film 116, 118, situated one on top of another, on a conveyer table 120. The mirror 114 is moved in accordance with commands from the controller to cut and seal pouches with the desired outline. This process has the advantage of having no fixed tooling and no limit on the pouch size and shape.

Fig. 25 illustrates the hot wheel sealing approach. The movement of the wheel 122 is guided by the controller to seal the films along outline 124. This method is extremely flexible and provides good control over temperature, time and pressure. It is similar to that which is presently used in vinyl fabrication.

Flexible outline weld tools that bend to the needed shapes as directed by an electronic signal may be employed. Another possibility is the use of rigid sections of outline weld tools that are selected to be combined with others to form customized outlines. If a limited number of custom outlines are required, a specific outline tool may be selected by the machine from a rack or rotary table as needed.

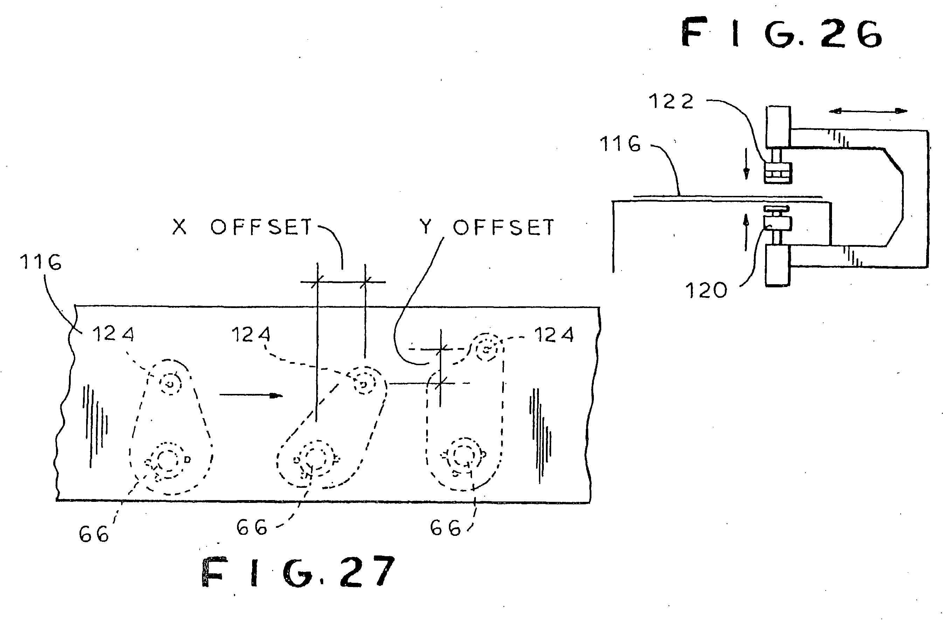

Prior to welding the pouch periphery, the attachment ring, tap and filter welds are made. Fig. 26 shows the welder itself, which includes a support 120 for the part, situated beneath the film 116 and a weld unit 122 supported above the film. The unit is moveable along an X-Y axis so as to position the parts as required relative to the film. Fig. 27 shows the film from above and illustrates that the attachment ring 66 and tap washer 124 may be aligned along the vertical axis of the pouch, offset along the X direction or offset along the Y direction, as illustrated.

In this regard, placement of the components could be achieved by robotic indexing machines. The center of the stoma receiving hole could be used as a reference. Variable index distance may be desireable.

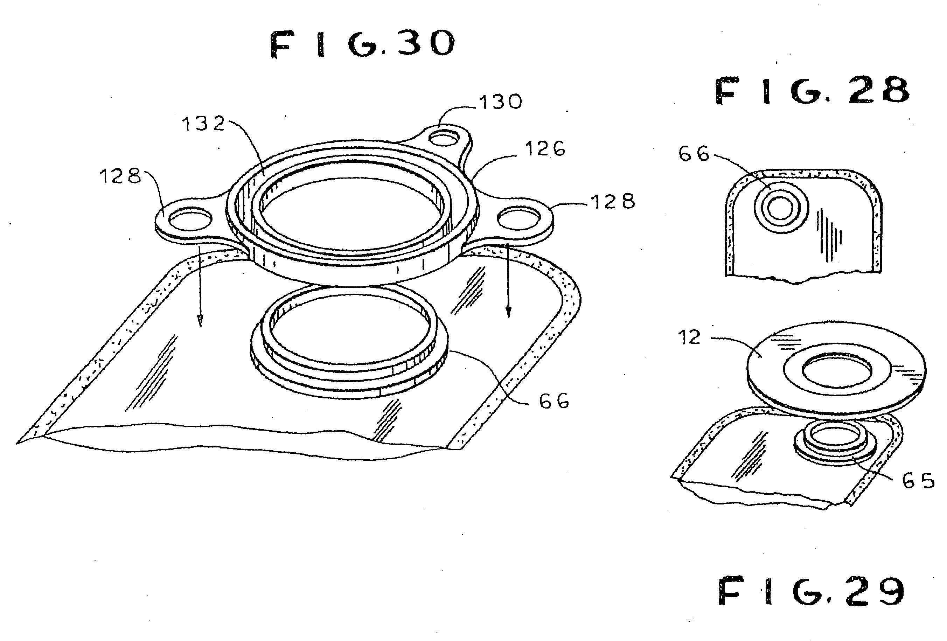

One of the features which is of concern in designing pouches is the "head room", which is the space between the attachment ring and the top edge of the pouch. It is usually desireable to minimize the head room, as shown in Fig. 28 which shows a pouch with the attachment toward the left side. This can be accomplished in the present invention by employing a single type of attachment ring which will act as a permanently welded base and which is designed to accept a variety of different components, depending upon whether the pouch is to be attached to a belt, is to be detachably coupled to the wafer or is to be non-detachably coupled to the wafer.

Figure 29 illustrates a pouch with an attachment ring 66 near the right side which is designed to directly receive a wafer 12 permanently affixed thereto. In this way, ring 66 can be used to form a one piece applicance.

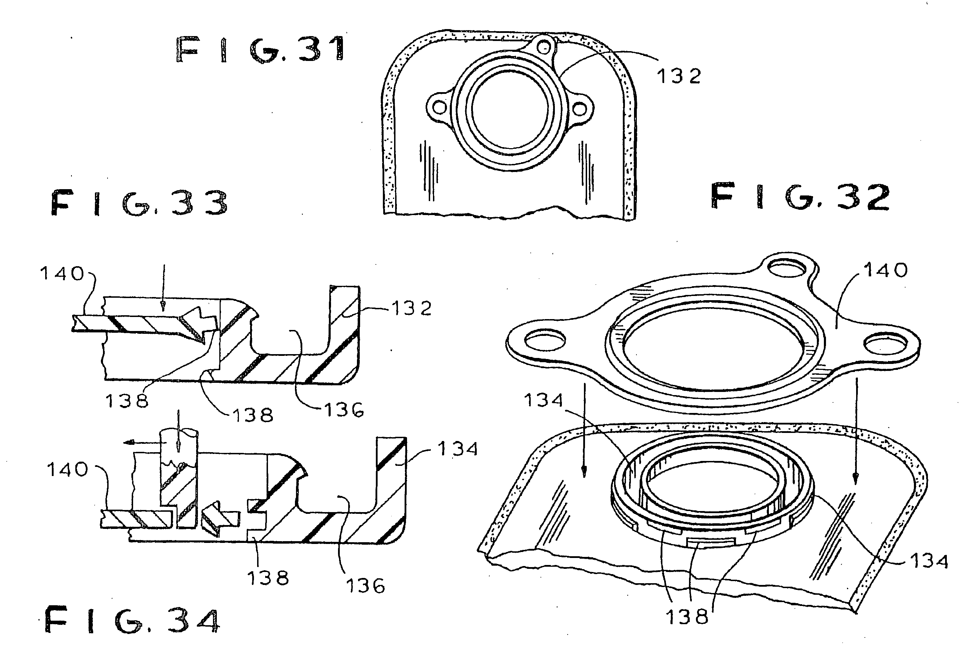

Figure 30 shows the same pouch and ring 66 but in this case a flange 126 is attached thereto. Flange 126 has belt receiving lugs 128 and a burp tab 130. Flanges of different configurations can be used for different applications. As illustrated, flange 126 has an annular channel 132 for detachable coupling with a mating pouch ring. Flange 132 can be welded to ring 66, as shown in Fig. 31. It can have an intergral channel 136 and snap-fit into place on a ring 134, as shown in Fig. 32.

Figs. 33 and 34 illustrate two different attachment ring cross-sections. Each ring 134 has a "U" shaped channel 136 with a series of spaced protrusions 138 on the exterior wall.

A flange 140 with lugs for belt attachment and/or a burp tab can be positioned over the pouch attachment ring in a snap-in fashion, where the projections on the exterior wall of the ring have a tapered structure, as shown in Fig. 33. Alternatively, a stretch and release method could be used where the resiliency of the plastic flange material is utilized, as indicated by the arrows in Fig. 34.

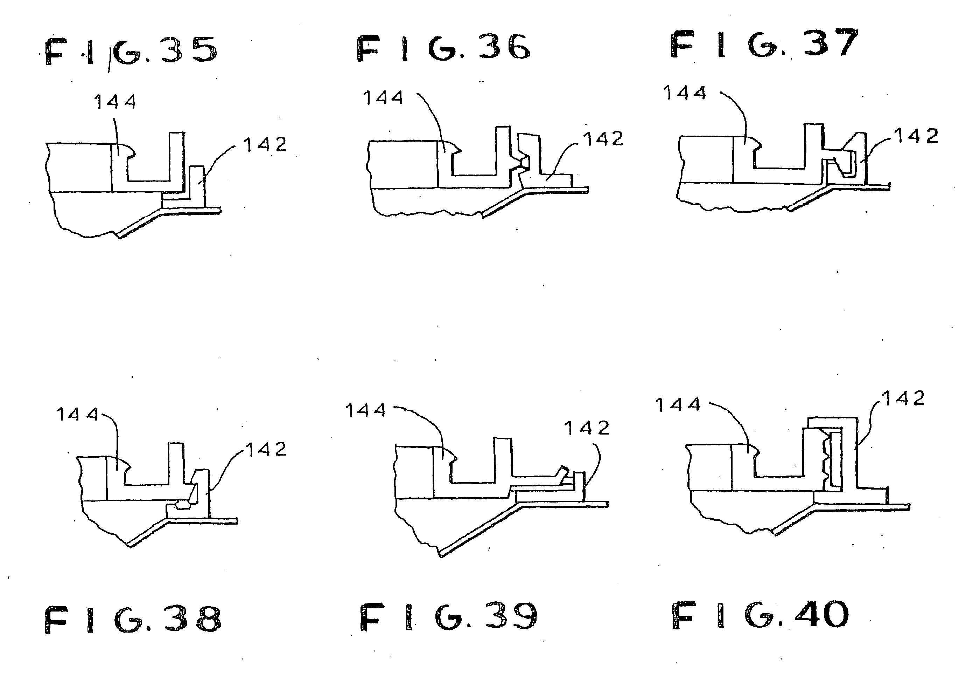

Another alternative is to employ a two part attachment ring, as illustrated in Figs. 35 through 40. Here a first part 142 of the ring is welded directly and permanently to the pouch wall and a second part 144 of the ring, which includes the annular channel, is joined to the first part by adhesive, as seen in Fig. 35, by a radially outwardly extending protrusion with a sealing ring, as shown in Fig. 36, by a "Ll" shaped protrusion, as shown in Fig. 37, a snap undercut structure, as shown in Fig. 38, a hook and loop ribbon fastener type attachment, as seen in Fig. 39 or a ribbed wall with a flat seal, as shown in Fig. 40.

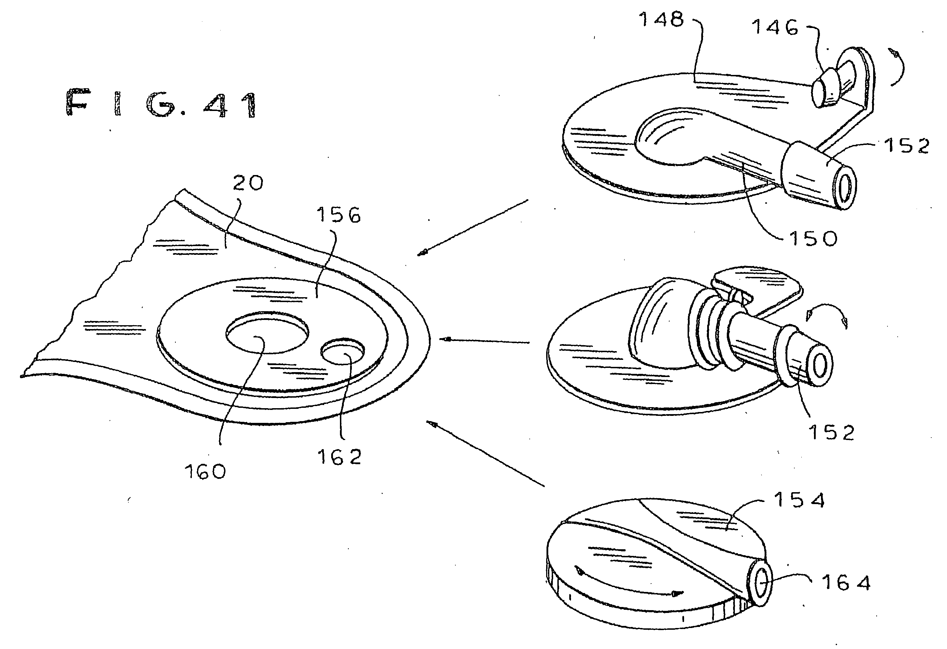

As mentioned above, the tap style can also be customized. Fig. 41 illustrates three different tap styles. At the top is shown the fold over type, where the plug 146 is affixed to the base 148. The flexible outlet 150 is provided with a tapered sleeve 152 to accept the plug 146 in a sealing manner. The middle illustrates the Accuseal® type, where the tube 152 is rotated to seal. The bottom illustrates the rotary type, where the base 154 is rotated between open and closed positions.

All three tap types can be mounted to the pouch using a unique weld tap washer 156. Washer 156 has first and second knock out slugs which are selectively removed to suit the desired tap style.

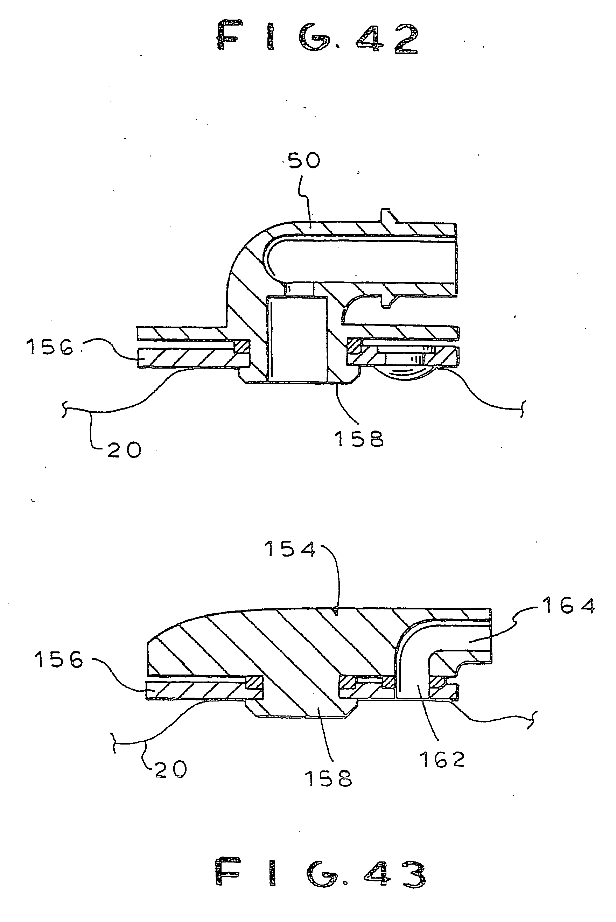

As seen in Figs. 42 and 43, each tap style has a central downward protruding post 158 which snap fits into the center opening 160 in washer 156. If the tap is of the foldover or Accuseal® type, post 158 is hollow and defines a conduit connecting the pouch interior with the tap tube, as seen in Fig. 42. The washer body covers the second opening 162 in the washer which is closed at the bottom by the pouch wall.

If a rotary type tap is employed, as seen in Fig. 43, the central post 158 is solid and acts to define the axis about which the tap rotates. The tube 164 terminates above the position of the second opening 162 and when in the appropriate rotational position, will align with the second opening so as to provide a channel to the pouch interior, as seen in Figure 43.

Certain pouches have no tap but instead an open bottom which is closed when in use by a tail clip. This feature can be customized by tailoring the length of the clip to match the width of the pouch outlet.

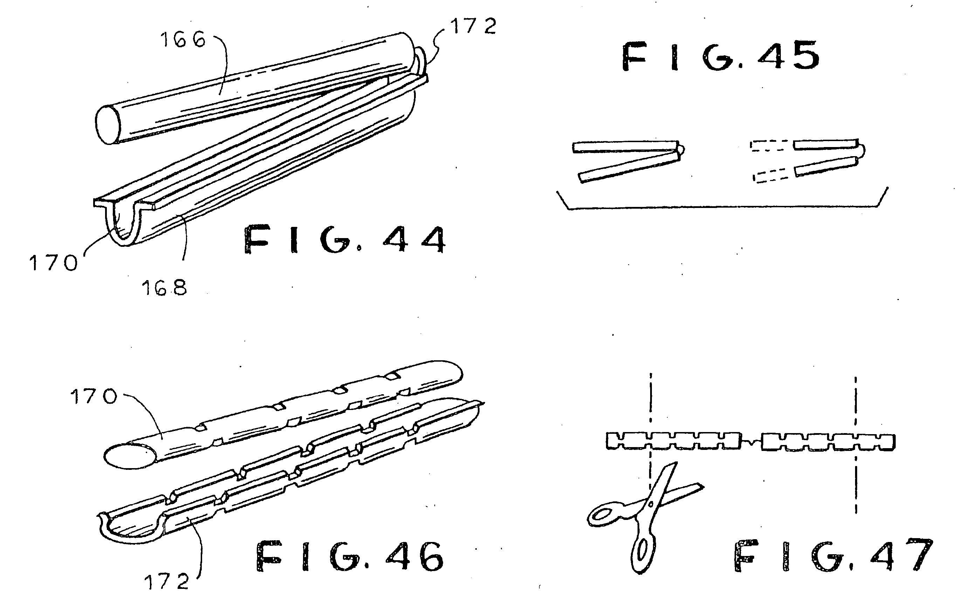

Fig. 44 shows a tail clip which consists of a bar 166 and a "U" shaped member 168 with a bar receiving channel 17C. The parts are attached to each other by a resilient part 172 which maintains alignment between the parts. The pouch tail is wrapped around bar 166 and the bar is then snapped into the member 168 to close the tail. The parts can be cut to the appropriate size, as seen in Fig. 45.

Fig. 46 shows a second embodiment where the bar 170 and receiving member 172 are notched to facilitate cutting both parts to equal lengths to match the tail width, as seen in Fig. 47.

It will now be appreciated that the present invention relates to fabrication of customized ostomy devices including wafers and pouches of either the one piece or two piece detachable type. Detailed measurements of the topography of the stoma area are taken and recorded. These measurements are used to form an electronic 3-dimensional image or map of the anatomy of the patient. This image, user history and preferences are maintained in a patient file databse.

When devices are to be produced, the information in the patient's file is downloaded into a computer which analyzes the information and selects the physical attributes of the wafer, including the adhesive type, stoma receiving opening size, shape and location, the outline and structure of the pouch, including the wall film, colar, tail location, and type of attachment ring, tab style, tab position and filter.

The computer then causes the fabrication equipment to select the appropriate materials and tools, to make the necessary adjustments and guide the tools to produce wafers and pouches to suit the specific requirements of the user. Accessory selection, packaging and shipping is also in accordance with the user's needs.

While only a limited number of emodiments of the present invention are disclosed for purposes of illustration, it is obvious that many variations and modifications could be made thereto. It is intended to cover all of these variations and modifications which fall within the scope of this invention, as set forth in the following claims:

## Claims

1. A method for fabricating customized ostomy devices including a wafer (12, 18) and a collection pouch (14, 20) wherein the wafer is an adhesive wafer and the method comprises the following steps: measuring the surface or topography of the stomal area of the patient; recording information relating to the measurements of the stoma area, transforming the recorded information into electronic form; utilizing the electronic information to select the physical attributes of the ostomy device including physical attributes for the wafer (12, 18), and fabricating the ostomy device to have physical attributes selected in accordance with the measurement information; and wherein the step of utilizing the electronic information comprises the step of selecting the surface contour of the adhesive wafer (12, 18) in accordance with said information and the step of fabricating the ostomy device comprises the step of forming the adhesive wafer (12, 18) with the selected surface contour separated from the pouch to have said physical attributes for the wafer.

2. The method of claim 1 **characterised in that** the step of utilizing the electronic information comprises the step of selecting the size, shape and location of the stoma receiving opening in the adhesive wafer (12, 18) in accordance with said information and the step of fabricating the ostomy devices comprises the step of forming the adhesive wafer (12, 18) to have a stoma receiving opening of the selected size, shape and location.

3. The method of claim 1 **characterised in that** the step of utilizing the electronic information comprises the step of selecting the size and shape of the adhesive wafer (12, 18) in accordance with said information and the step of fabricating the ostomy devices comprises the step of forming the outline of adhesive wafer (12, 18) in accordance with the selected size and shape.

4. The method of claim 1 **characterised in that** the step of utilizing the electronic information comprises the step of selecting the size and shape of the pouch (14, 20) in accordance with said information and the step of fabricating the ostomy device comprises the step of forming the pouch (14, 20) having the selected size and shape.

5. The method of claim 1 **characterised in that** the step of utilizing the electronic information comprises the step of selecting the location of the attachment ring in the pouch wall in accordance with said information and the step of fabricating the ostomy device comprises the step of forming the pouch (14, 20) with the attachment ring positioned at the selected location.

6. The method of claim 1 **characterised in that** the step of utilizing the electronic information comprises the step of selecting the location and type of tap (146, 150, 152, 154, 164) in accordance with said information and the step of fabricating the ostomy device comprises the step of forming the pouch with the tap (146, 150, 152, 154, 164) at the selected location and of the selected type.

7. The method of claim 1 **characterised in that** it further comprises the step of obtaining information as to the patient history and preferences and transforming same into electronic form.

8. The method of claim 7 **characterised in that** the step of utilizing the electronic information comprises the step of selecting the type of adhesive to be used in the wafer in accordance with said information and the step of fabricating the ostomy devices comprises the step of applying adhesive of the selected type on a backing to form the adhesive wafer (12, 18).

9. The method of claim 7 charactgerised in that the step of utilizing the electronic information comprises the step of selecting a barrier film in accordance with said information and the step of fabricating the ostomy device comprises the step of forming the pouch (14, 20) with walls composed of the selected barrier film.

10. The method of claim 7 **characterised in that** the step of utilizing the electronic information comprises the step of selecting a barrier film color in accordance with said information and the step of fabricating the ostomy device comprises the step of forming the pouch (14, 20) with walls comprised of barrier film with the selected color.

11. The method of claim 7 **characterised in that** the step of utilizing the electronic information comprises the step of selecting the appropriate accessories to be supplied with the ostomy device in accordance with said information and further comprising the step of packaging the selected accessories with the ostomy device.

12. The method of claim 7 **characterised in that** the step of utilizing the electronic information comprises the step of selecting whether the pouch (14, 20) is to be detachably coupled to the adhesive wafer (12, 18) in accordance with said information and the step of fabricating the ostomy device comprises the step of forming the ostomy device with means for detachably coupling the pouch to the wafer, if same is selected.

13. The method of claim 1 **characterised in that** the step of measuring the topography of the stomal area comprises the steps of scanning of the stomal area with a laser.

14. The method of claim 1 **characterised in that** the step of measuring the topography of the stomal area comprises the step of forming a mold of the stomal area.

15. The method of claim 14 **characterised in that** the step of forming a mold comprises the steps of filling a collar (46) with mold material (52), placing the filled collar (46) adjacent the stomal area and allowing the mold material (52) to solidify.

16. The method of claim 16 **characterised in that** the step of measuring the topography of the stoma area further comprises the step of scanning the surface of solidified mold material (52) with a laser.

17. The method of claim 3 **characterised in that** the step of forming the outline of the adhesive wafer (12, 18) comprises the steps of selecting with a wafer with a size which is larger than the selected size and cutting the wafer to have an outline in accordance with the selected size and shape.

18. The method of claim 1 **characterised in that** the step of forming the adhesive wafer with the selected surface contour comprises the steps of selecting a convex disk with a central opening, affixing the convex disk on the wafer and coating the convex surface of the disk with adhesive.

19. The method of claim 1 **characterised in that** the step of forming the adhesive wafer with the selected surface contour comprises the steps of heating an adhesive layer and forming the heated adhesive layer into a mold.

20. The method of claim 18 **characterised in that** it further comprises the step of utilizing a filler material as a back for said adhesive layer.

21. The method of claim 1 **characterised in that** the step of forming the adhesive wafer with the selected surface contour comprises forming a plurality of slices (71-81) each with a portion of the selected surface contour, joining the slices (71-81) to form a base and depositing a layer of adhesive over said base.

22. The method of claim 18 **characterised in that** it further comprises the step of selecting a convex insert and snap-fitting the convex insert into the central opening of the convex disk (86).

23. The method of claim 18 **characterised in that** it further comprises the steps of affixing a annular enclosure defining an internal chamber (88) over the convex disk (86) surface prior to coating same with adhesive (64).

24. The method of claim 23 **characterised in that** it further comprises the step of introducing a contour altering material (96) into the chamber (88).

25. The method of claim 24 **characterised in that** it further comprises the step of manipulating the material to alter the contour of the wafer.

26. The method of claim 24 **characterised in that** the material comprises corn starch.

27. The method of claim 24 **characterised in that** the material comprises a fluid.

28. The method of claim 24 **characterised in that** the material comprises a quick setting resin.

29. The method of claim 24 **characterised in that** the material comprises a compressed sponge and further comprising the step of introducing water into the chamber to expand the sponge (96).

30. The method of claim 4 **characterised in that** the step of forming the pouch comprises the steps of placing sheets of barrier film one on top of the other, welding the surface of one sheet to the surface of the other sheet (106) by guiding a welding instrument along an outline determined in accordance with the recorded information to form the pouch periphery.

31. The method of claim 30 **characterised in that** the welding instrument comprises a laser beam and wherein the step of guiding comprises directing the beam onto the sheets.

32. The method of claim 30 **characterised in that** the welding instrument comprises a hot wheel.

33. The method of claim 5 **characterised in that** it further comprises the step of affixing the wafer to the attachment ring.

34. The method of claim 33 **characterised in that** it further comprises the step of affixing a first coupling ring to said attachment ring and affixing a second coupling ring to the wafer.

35. The method of claim 1 **characterised in that** the step of recording the measurement information comprises the step of marking a card to indicate various aspects of the topography of the stomal area.

36. The method of claim 35 **characterised in that** the step of transforming the recorded information into electronic form comprises the step of converting the information into digital form.

37. The method of claim 1 **characterised in that** the step of recording the measurement information comprises the step of forming simulated parts of the wafer, altering the simulated parts in accordance with the measurements, and assembling the altered parts in a manner indicative of the desired relationship between the parts.

38. The method of claim 37 **characterised in that** the step of transforming the recorded information comprises the step of scanning the surface of the assembled parts and converting the scanned information into digital form.

39. The method of claim 1 **characterised in that** the customized ostomy devices include a stoma receiving opening in the adhesive wafer (12, 18), an attachment ring (65) (66) defining the area in which said opening is situated, a waste collection pouch (14, 20) with an inlet, and means for mounting said pouch on said wafer with said inlet aligned with said opening, wherein the size, shape and location of said opening within said area is selected in accordance with the size, shape and location of the stoma of the user.

40. The method of claim 39 **characterised in that** said wafer (12, 18) has a shape and size selected in accordance with the topography of the stomal area of the user.

41. The method of claim 39 **characterised in that** the pouch (14, 20) has a contour selected in accordance with the topography of the stomal area of the user.

42. The method of claim 39 **characterised in that** said pouch (14, 20) comprises an attachment ring and wherein the location of said attachment ring on said pouch is selected in accordance with the topography of the stomal area of the user.

43. The method of claim 39 **characterised in that** the surface of contour of said wafer is selected in accordance with the topography of the stomal area of the user.

44. The method of claim 1 **characterised in that** the customized ostomy devices include a stoma receiving opening in the adhesive wafers (12, 18), an inlet in the collection pouch, and means for attaching said wafer (12, 18) and said pouch (14, 20) with said opening aligned with said inlet, said attaching means comprising a first attachment ring welded to said pouch, a flange (126) removably mounted on said first ring and a second attachment ring welded to said wafer (12, 18), one of said first ring and said flange (126) comprising an annular channel (132), said second ring comprising an annular protrusion adapted to be removeably received within said channel (132).

45. The method of claim 44 **characterised in that** said first ring has an exterior wall, the device further comprising protrusions (138) on said wall spaced to receive said flange (126) therebetween.

46. The method of claim 45 **characterised in that** said flange is formed of material flexible enough to pass over the protrusions (138) in one direction.

47. The method of claim 44 **characterised in that** the device further comprises means for removeably mounting said flange (126) on said first ring.

48. The method of claim 47 **characterised in that** said removeable mounting means comprises a recess in said first ring and a protrusion on said flange (126) adapted to be received within said recess.

49. The method of claim 1 **characterised in that** the customized ostomy device includes means for mounting said pouch (14, 20) on said wafer (12, 18), and a tap mounting washer (156) affixed to a wall of said pouch (14, 20) over an opening in said wall, said washer (156) comprising a central opening (160) aligned with said pouch opening, a second opening (162) offset from said central opening, a removeable disk in said second opening (162), and a tap comprising a base with a central post (158) adapted to be received within said central opening (160) in said washer (156).

50. The method of claim 49 **characterised in that** said post (158) is hollow and acts to connect said tap to the interior of said pouch (14, 20).

51. The method of claim 49 **characterised in that** said post (158) is solid and acts as an axis as the base is rotated relative to said washer (156).

52. The method of claim 51 **characterised in that** said tap comprises a conduit and **in that** said conduit terminates in said base so as to align with said second opening (162) when said base is in a selected rotational position relative to said washer (156).

53. The method of claim 1 **characterised in that** the device includes a tail clip comprising first and second interengaging parts (166, 168, 170, 172), each of said parts being notched at regular intervals to facilitate trimming of each to substantially the same length.

## Patentansprüche

1. Verfahren zum Herstellen kundenspezifischer Ostomievorrichtungen, die eine Scheibe (12, 18) und einen Sammelbeutel (14, 20) umfassen, wobei die Scheibe eine Klebstoffscheibe ist und das Verfahren die folgenden Schritte aufweist: Messen der Oberfläche oder Topographie des Stomabereichs des Patienten; Aufzeichnen von Informationen, die die Messungen des Stomabereichs betreffen; Transformieren der aufgezeichneten Informationen in elektronische Form; Nutzen der elektronischen Informationen, um die physikalischen Merkmale der Ostomievorrichtung auszuwählen einschließlich physikalischer Merkmale für die Scheibe (12, 18); und Herstellen der Ostomievorrichtung, so dass sie physikalische Merkmale hat, die entsprechend den Messinformationen ausgewählt sind; und wobei der Schritt Nutzen der elektronischen Information den Schritt Auswählen des Oberflächenumrisses der Klebstoffscheibe (12, 18) entsprechend dieser Information aufweist und der Schritt Herstellen der Ostomievorrichtung den Schritt aufweist Ausbilden der Klebstoffscheibe (12, 18) mit dem ausgewählten Oberflächenumriss getrennt vom Beutel, um die physikalischen Merkmale für die Scheibe zu haben.

2. Verfahren nach Anspruch 1, **dadurch gekennzeichnet, dass** der Schritt Nutzen der elektronischen Informationen den Schritt Auswählen der Größe, der Form und des Orts der stomaaufnehmenden Öffnung in der Klebstoffscheibe (12, 18) entsprechend dieser Informationen aufweist, und dass der Schritt Herstellen der Ostomievorrichtungen den Schritt Ausbilden der Klebstoffscheibe (12, 18) mit einer stomaaufnehmenden Öffnung mit der ausgewählten Größe, Form und an dem ausgewählten Ort aufweist.

3. Verfahren nach Anspruch 1, **dadurch gekennzeichnet, dass** der Schritt Nutzen der elektronischen Informationen den Schritt Auswählen der Größe und Form der Klebstoffscheibe (12, 18) entsprechend dieser Informationen aufweist, und dass der Schritt Herstellen der Ostomievorrichtungen den Schritt Ausbilden des Umrisses der Klebstoffscheibe (12, 18) entsprechend der ausgewählten Größe und Form aufweist.

4. Verfahren nach Anspruch 1, **dadurch gekennzeichnet, dass** der Schritt Nutzen der elektronischen Informationen den Schritt Auswählen der Größe und Form des Beutels (14, 20) entsprechend dieser Informationen aufweist, und dass der Schritt Herstellen der Ostomievorrichtung den Schritt Ausbilden des Beutels (14, 20) mit der ausgewählten Größe und Form aufweist.

5. Verfahren nach Anspruch 1, **dadurch gekennzeichnet, dass** der Schritt Nutzen der elektronischen Informationen den Schritt Auswählen des Orts für den Befestigungsring in der Beutelwand entsprechend dieser Informationen aufweist, und dass der Schritt Herstellen der Ostomievorrichtung den Schritt Ausbilden des Beutels (14, 20) aufweist, wobei der Befestigungsring an der ausgewählten Stelle positioniert ist.

6. Verfahren nach Anspruch 1, **dadurch gekennzeichnet, dass** der Schritt Nutzen der elektronischen Informationen den Schritt Auswählen des Orts und der Art des Hahns (146, 150, 152, 154, 164) entsprechend dieser Informationen aufweist, und dass der Schritt Herstellen der Ostomievorrichtung den Schritt Ausbilden des Beutels mit dem Hahn (146, 150, 152, 154, 164) von der ausgewählten Art und an der ausgewählten Stelle aufweist.

7. Verfahren nach Anspruch 1, **dadurch gekennzeichnet, dass** es ferner den Schritt Erhalten der die Patientengeschichte und die Vorlieben betreffenden Information und Transformieren dieser in elektronische Form aufweist.

8. Verfahren nach Anspruch 7, **dadurch gekennzeichnet, dass** der Schritt Nutzen der elektronischen Information den Schritt Auswählen der Art des in der Scheibe zu verwendenden Klebstoffs entsprechend dieser Information aufweist, und dass der Schritt Herstellen der Ostomievorrichtungen den Schritt Auftragen von Klebstoff der ausgewählten Art auf einem Träger aufweist, um die Klebstoffscheibe (12, 18) zu bilden.

9. Verfahren nach Anspruch 7, **dadurch gekennzeichnet, dass** der Schritt Nutzen der elektronischen Information den Schritt Auswählen einer Sperrfolie entsprechend dieser Information aufweist, und dass der Schritt Herstellen der Ostomievorrichtung den Schritt Ausbilden des Beutels (14, 20) mit Wänden aufweist, welche aus der ausgewählten Sperrfolie bestehen.

10. Verfahren nach Anspruch 7, **dadurch gekennzeichnet, dass** der Schritt Nutzen der elektronischen Information den Schritt Auswählen einer Sperrfolienfarbe entsprechend dieser Information aufweist, und dass der Schritt Herstellen der Ostomievorrichtung den Schritt Ausbilden des Beutels (14, 20) mit Wänden aufweist, die aus Sperrfolie mit der ausgewählten Farbe bestehen.

11. Verfahren nach Anspruch 7, **dadurch gekennzeichnet, dass** der Schritt Nutzen der elektronischen Information den Schritt Auswählen der passenden mit der Ostomievorrichtung zu liefernden Zubehöre entsprechend dieser Information aufweist, und dass es ferner den Schritt Verpacken der ausgewählten Zubehöre mit der Ostomievorrichtung aufweist.

12. Verfahren nach Anspruch 7, **dadurch gekennzeichnet, dass** der Schritt Nutzen der elektronischen Information den Auswahlschritt entsprechend dieser Information aufweist, ob der Beutel (14, 20) abnehmbar mit der Klebstoffscheibe (12, 18) verbunden werden soll, und dass der Schritt Herstellen der Ostomievorrichtung den Schritt Ausbilden der Ostomievorrichtung mit einer Einrichtung zum abnehmbaren Verbinden des Beutels mit der Scheibe aufweist, wenn dieses gewählt wird.

13. Verfahren nach Anspruch 1, **dadurch gekennzeichnet, dass** der Schritt Messen der Topographie des Stomabereichs den Schritt Abtasten des Stomabereichs mit einem Laser aufweist.

14. Verfahren nach Anspruch 1, **dadurch gekennzeichnet, dass** der Schritt Messen der Topographie des Stomabereichs den Schritt Herstellen einer Form des Stomabereichs aufweist.

15. Verfahren nach Anspruch 14, **dadurch gekennzeichnet, dass** der Schritt Herstellen einer Form die Schritte Füllen einer Manschette (46) mit Formmaterial (52), Plazieren der gefüllten Manschette (46) benachbart zum Stomabereich und Verfestigenlassen des Formmaterials (52) aufweist.

16. Verfahren nach Anspruch 14, **dadurch gekennzeichnet, dass** der Schritt Messen der Topographie des Stomabereichs ferner den Schritt Abtasten der Oberfläche des verfestigten Formmaterials (52) mit einem Laser aufweist.

17. Verfahren nach Anspruch 3, **dadurch gekennzeichnet, dass** der Schritt Ausbilden des Umrisses der Klebstoffscheibe (12, 18) die Schritte Auswählen einer Scheibe mit einer größeren Größe als der ausgewählten Größe und Schneiden der Scheibe, so dass sie einen der ausgewählten Größe und Form entsprechenden Umriss hat, aufweist.

18. Verfahren nach Anspruch 1, **dadurch gekennzeichnet, dass** der Schritt Ausbilden der Klebstoffscheibe mit dem ausgewählten Oberflächenumriss die Schritte Auswählen einer konvexen Platte mit einer Mittelöffnung, Befestigen der konvexen Platte auf der Scheibe und Beschichten der konvexen Oberfläche der Platte mit Klebstoff aufweist.

19. Verfahren nach Anspruch 1, **dadurch gekennzeichnet, dass** der Schritt Ausbilden der Klebstoffscheibe mit dem ausgewählten Oberflächenumriss die Schritte Erwärmen einer Klebstoffschicht und Formen der erwärmten Klebstoffschicht in eine Form aufweist.

20. Verfahren nach Anspruch 19, **dadurch gekennzeichnet, dass** es ferner den Schritt Verwenden eines Füllmaterials als eine Verstärkung für die Klebstoffschicht aufweist.

21. Verfahren nach Anspruch 1, **dadurch gekennzeichnet, dass** der Schritt Ausbilden der Klebstoffscheibe mit dem ausgewählten Oberflächenumriss aufweist: das Ausbilden einer Vielzahl von Scheiben (71 - 81) jeweils mit einem Teil des ausgewählten Oberflächenumrisses, Verbinden der Scheiben (71 - 81), um eine Unterlage zu bilden, und Abscheiden einer Klebstoffschicht über dieser Unterlage.

22. Verfahren nach Anspruch 18, **dadurch gekennzeichnet, dass** es ferner den Schritt Auswählen eines konvexen Einsatzes und Druckeinpassen des konvexen Einsatzes in der Mittelöffnung der konvexen Platte (86) aufweist.

23. Verfahren nach Anspruch 18, **dadurch gekennzeichnet, dass** es ferner den Schritt aufweist: Befestigen eines ringförmigen eine innere Kammer (88) begrenzenden Gehäuses, über der Oberfläche der konvexen Platte (86), bevor diese mit Klebstoff (64) beschichtet wird.

24. Verfahren nach Anspruch 23, **dadurch gekennzeichnet, dass** es ferner den Schritt Einführen eines umrissverändernden Materials (96) in die Kammer (88) aufweist.

25. Verfahren nach Anspruch 24, **dadurch gekennzeichnet, dass** es ferner den Schritt Manipulieren des Materials aufweist, um den Umriss der Scheibe zu verändern.

26. Verfahren nach Anspruch 24, **dadurch gekennzeichnet, dass** das Material Maisstärke aufweist.

27. Verfahren nach Anspruch 24, **dadurch gekennzeichnet, dass** das Material ein Fluid aufweist.

28. Verfahren nach Anspruch 24, **dadurch gekennzeichnet, dass** das Material ein schnell härtendes Kunstharz aufweist.

29. Verfahren nach Anspruch 24, **dadurch gekennzeichnet, dass** das Material einen zusammengedrückten Schwamm aufweist und dass es ferner den Schritt Einführen von Wasser in die Kammer, um den Schwamm (96) auszudehnen, aufweist.

30. Verfahren nach Anspruch 4, **dadurch gekennzeichnet, dass** der Schritt Ausbilden des Beutels die folgenden Schritte aufweist: Plazieren von Sperrfolienschichten übereinander, Schweißen der Oberfläche einer Schicht an die Oberfläche der anderen Schicht (106) durch Führen eines Schweißgeräts entlang eines Umrisses, welcher entsprechend der aufgezeichneten Information bestimmt wird, um den Beutelumfang auszubilden.

31. Verfahren nach Anspruch 30, **dadurch gekennzeichnet, dass** das Schweißgerät einen Laserstrahl aufweist, und wobei der Führungsschritt das Lenken des Strahls auf die Schichten aufweist.

32. Verfahren nach Anspruch 30, **dadurch gekennzeichnet, dass** das Schweißgerät eine heiße Rolle aufweist.

33. Verfahren nach Anspruch 5, **dadurch gekennzeichnet, dass** es ferner den Schritt Befestigen der Scheibe an dem Befestigungsring aufweist.

34. Verfahren nach Anspruch 33, **dadurch gekennzeichnet, dass** es ferner den Schritt Befestigen eines ersten Verbindungsrings an dem Befestigungsring und Befestigen eines zweiten Verbindungsrings an der Scheibe aufweist.

35. Verfahren nach Anspruch 1, **dadurch gekennzeichnet, dass** der Schritt Aufzeichnen der Messinformationen den Schritt Markieren einer Karte, um verschiedene Aspekte der Topographie des Stomabereichs anzuzeigen, aufweist.

36. Verfahren nach Anspruch 35, **dadurch gekennzeichnet, dass** der Schritt Transformieren der aufgezeichneten Informationen in elektronische Form den Schritt Umwandeln der Informationen in digitale Form aufweist.

37. Verfahren nach Anspruch 1, **dadurch gekennzeichnet, dass** der Schritt Aufzeichnen der Messinformationen den Schritt Herstellen simulierter Teile der Scheibe, Verändern der simulierten Teile entsprechend den Messungen und Montieren der veränderten Teile auf eine Weise, die das gewünschte Verhältnis zwischen den Teilen anzeigt, aufweist.

38. Verfahren nach Anspruch 37, **dadurch gekennzeichnet, dass** der Schritt Transformieren der aufgezeichneten Informationen den Schritt Abtasten der Oberfläche der montierten Teile und Umwandeln der abgetasteten Information in digitale Form aufweist.

39. Verfahren nach Anspruch 1, **dadurch gekennzeichnet, dass** die kundenspezifischen Ostomievorrichtungen aufweisen: eine stomaaufnehmende Öffnung in der Klebstoffscheibe (12, 18), einen Befestigungsring (65) (66), der den Bereich definiert, in dem die genannte Öffnung angeordnet ist, einen Abfallprodukt-Sammelbeutel (14, 20) mit einem Einlass und einer Einrichtung zum Montieren des Beutels an die Scheibe, wobei der Einlass mit der Öffnung ausgerichtet ist, wobei die Größe, Form und der Ort der Öffnung innerhalb dieses Bereichs entsprechend der Größe, der Form und des Orts des Stomas des Benutzers ausgewählt sind.

40. Verfahren nach Anspruch 39, **dadurch gekennzeichnet, dass** die Scheibe (12, 18) eine Form und Größe hat, die entsprechend der Topographie des Stomabereichs des Benutzers ausgewählt werden.

41. Verfahren nach Anspruch 39, **dadurch gekennzeichnet, dass** der Beutel (14, 20) einen Umriss hat, der entsprechend der Topographie des Stomabereichs des Benutzers ausgewählt wird.

42. Verfahren nach Anspruch 39, **dadurch gekennzeichnet, dass** der Beutel einen Befestigungsring aufweist, wobei der Ort des Befestigungsrings auf dem Beutel entsprechend der Topographie des Stomabereichs des Benutzers ausgewählt wird.

43. Verfahren nach Anspruch 39, **dadurch gekennzeichnet, dass** die Umrissoberfläche der Scheibe entsprechend der Topographie des Stomabereichs des Benutzers ausgewählt wird.

44. Verfahren nach Anspruch 1, **dadurch gekennzeichnet, dass** die kundenspezifischen Ostomievorrichtungen aufweisen: eine stomaaufnehmende Öffnung in der Klebstoffscheibe (12, 18), einen Einlass in dem Sammelbeutel und eine Einrichtung zum Befestigen der Scheibe (12, 18) und des Beutels (14, 20), wobei die Öffnung mit dem Auslass ausgerichtet ist, wobei die Befestigungseinrichtung einen an den Beutel geschweißten ersten Befestigungsring, einen abnehmbar auf den ersten Ring montierten Flansch (126) und einen an die Scheibe (12, 18) geschweißten zweiten Befestigungsring aufweist, wobei entweder der erste Ring oder der Flansch (126) einen ringförmigen Kanal (132) aufweisen, wobei der zweite Ring einen ringförmigen Vorsprung aufweist, der geeignet ist, abnehmbar in dem Kanal (132) aufgenommen zu werden.

45. Verfahren nach Anspruch 44, **dadurch gekennzeichnet, dass** der erste Ring eine Außenwand hat und die Vorrichtung ferner beabstandete Vorsprünge (138) auf der Wand aufweist, um den Flansch (126) dazwischen aufzunehmen.

46. Verfahren nach Anspruch 45, **dadurch gekennzeichnet, dass** der Flansch aus Material hergestellt wird, das flexibel genug ist, um in einer Richtung über die Vorsprünge (138) zu gehen.

47. Verfahren nach Anspruch 44, **dadurch gekennzeichnet, dass** die Vorrichtung ferner eine Einrichtung zum abnehmbaren Montieren des Flansches (126) auf dem ersten Ring aufweist.

48. Verfahren nach Anspruch 47, **dadurch gekennzeichnet, dass** die abnehmbare Montageeinrichtung eine Aussparung in dem ersten Ring und einen Vorsprung auf dem Flansch (126) aufweist, der geeignet ist, in der Aussparung aufgenommen zu werden.

49. Verfahren nach Anspruch 1, **dadurch gekennzeichnet, dass** die kundenspezifische Ostomievorrichtung aufweist: eine Einrichtung zum Montieren des Beutels (14, 20) auf der Scheibe (12, 18) und eine Hahnmontage-Dichtungsscheibe (156), die über einer Öffnung in der Wand an einer Wand des Beutels (14, 20) befestigt ist, wobei die Dichtungsscheibe (156) aufweist: eine mit der Beutelöffnung ausgerichtete Mittelöffnung (160), eine gegen die Mittelöffnung versetzte zweite Öffnung (162), eine entfernbare Platte in der zweiten Öffnung (162) und einen Hahn, der einen Sockel mit einem mittleren Pfosten (158) aufweist, welcher geeignet ist, in der Mittelöffnung (160) in der Dichtungsscheibe (156) aufgenommen zu werden.

50. Verfahren nach Anspruch 49, **dadurch gekennzeichnet, dass** der Pfosten (158) hohl ist und derart wirkt, dass er den Hahn mit dem Inneren des Beutels (14, 20) verbindet.

51. Verfahren nach Anspruch 49, **dadurch gekennzeichnet, dass** der Pfosten (158) massiv ist und als eine Achse fungiert, wenn der Sockel relativ zu der Dichtungsscheibe (156) gedreht wird.

52. Verfahren nach Anspruch 51, **dadurch gekennzeichnet, dass** der Hahn eine Rohrleitung aufweist, und **dadurch**, dass diese Rohrleitung in dem Sockel endet, so dass sie mit der zweiten Öffnung (162) ausgerichtet ist, wenn der Sockel in einer ausgewählten Drehposition relativ zu der Dichtungsscheibe (156) ist.

53. Verfahren nach Anspruch 1, **dadurch gekennzeichnet, dass** die Vorrichtung eine Abschlussklemme aufweist, die erste und zweite ineinandergreifende Teile (166, 168, 170, 172) aufweist, welche in regelmäßigen Intervallen eingekerbt sind, um jeweils das Zuschneiden auf im wesentlichen die gleiche Länge zu erleichtern.

## Revendications

1. Procédé de fabrication de dispositifs d'ostomie personnalisés comprenant une pastille (12, 18) et une poche de recueil (14, 20), où la pastille est une pastille adhésive et le procédé comporte les étapes suivantes: mesurer la surface ou la topographie de la zone stomale du patient; enregistrer les informations se rapportant aux mesures de la zone stomale, transformer les informations enregistrées sous forme électronique; utiliser les informations électroniques pour sélectionner les attributs physiques du dispositif d'ostomie incluant les attributs physiques pour la pastille (12, 18) et fabriquer le dispositif de stomie pour avoir les attributs physiques sélectionnés en conformité avec les informations de mesure; et où l'étape d'utilisation des informations électroniques comprend l'étape de sélection du contour de surface de la pastille adhésive (12, 18) en conformité avec lesdites informations, et l'étape de fabrication du dispositif d'ostomie comprend l'étape de la formation de la pastille adhésive (12, 18) avec le contour de surface sélectionné séparé de la poche pour avoir lesdits attributs physiques pour la pastille.

2. Procédé selon la revendication 1, **caractérisé en ce \que** l'étape d'utilisation des informations électroniques comprend l'étape consistant à sélectionner la dimension, la forme et l'emplacement de l'ouverture de réception de stoma dans la pastille adhésive (12, 18) en conformité avec lesdites informations et l'étape consistant à fabriquer les dispositifs d'ostomie comprend l'étape de formation de la pastille adhésive (12, 18) pour avoir une ouverture de réception de stomie de la dimension, forme et emplacement sélectionnés.

3. Procédé selon la revendication 1, **caractérisé en ce que** l'étape consistant à utiliser les informations électroniques comporte l'étape de sélection de la dimension et de la forme de la pastille adhésive (12, 18) en conformité avec lesdites informations et l'étape de fabrication des dispositifs d'ostomie comporte l'étape de formation du contour de la pastille adhésive (12, 18) en conformité avec la dimension et forme sélectionnées.

4. Procédé selon la revendication 1, **caractérisé en ce que** l'étape d'utilisation des informations électroniques comporte l'étape de sélection de la dimension et de la forme de la poche (14, 20) en conformité avec lesdites informations et l'étape de fabrication du dispositif d'ostomie comporte l'étape de formation de la poche (14, 20) ayant la dimension et la forme sélectionnées.

5. Procédé selon la revendication 1, **caractérisé en ce que** l'étape d'utilisation des informations électroniques comporte l'étape de sélection de l'emplacement de la bague de fixation dans la paroi de poche en conformité avec lesdites informations, et l'étape de fabrication du dispositif d'ostomie comporte l'étape de formation de la poche (14, 20) avec la bague de fixation positionnée à l'emplacement sélectionné.

6. Procédé selon la revendication 1, **caractérisé en ce que** l'étape d'utilisation des informations électroniques comporte l'étape de sélection de l'emplacement et du type d'une coulée (146, 150, 152, 154, 164) en conformité avec lesdites informations et l'étape de fabrication du dispositif d'ostomie comporte l'étape de formation de la poche avec la coulée (146, 150, 152, 154, 164) à l'emplacement sélectionné et du type sélectionné.

7. Procédé selon la revendication 1, **caractérisé en ce qu'**il comporte, en outre, l'étape d'obtention d'informations concernant l'histoire du patient et les préférences du patient et la transformation de ceux-ci sous forme électronique.

8. Procédé selon la revendication 7, **caractérisé en ce que** l'étape d'utilisation des informations électroniques comporte l'étape de sélection du type d'adhésif à utiliser dans la pastille en conformité avec lesdites informations et l'étape de fabrication des dispositifs d'ostomie comportent l'étape d'application d'un adhésif du type sélectionné sur un renfort afin de former la pastille adhésive (12, 18).

9. Procédé selon la revendication 7, **caractérisé en ce que** l'étape d'utilisation des informations électroniques comporte l'étape de sélection d'un film barrière en conformité avec lesdites informations et l'étape de fabrication des dispositifs d'ostomie comporte l'étape de formation de la poche (14, 20) avec des parois composées du film barrière sélectionné.

10. Procédé selon la revendication 7, **caractérisé en ce que** l'étape d'utilisation des informations électroniques comporte l'étape de sélection d'une couleur de film barrière en conformité avec lesdites informations et l'étape de fabrication du dispositif d'ostomie comporte l'étape de formation de la poche (14, 20) avec des parois constituées du film barrière avec la couleur sélectionnée.

11. Procédé selon la revendication 7, **caractérisé en ce que** l'étape d'utilisation des informations électroniques comporte l'étape de sélection des accessoires appropriés à délivrer au dispositif d'ostomie en conformité avec lesdites informations et comprenant, en outre, l'étape de conditionnement des accessoires sélectionnés avec le dispositif d'ostomie.

12. Procédé selon la revendication 7, **caractérisé en ce que** l'étape d'utilisation des informations électroniques comporte l'étape de sélection consistant à savoir si la poche (14, 20) doit être couplée de manière détachable à la pastille (12, 18) en conformité avec lesdites informations et l'étape de fabrication du dispositif d'ostomie comporte l'étape de formation du dispositif d'ostomie avec des moyens pour le couplage, de manière détachable, de la poche à la pastille, si celle-ci est sélectionnée.

13. Procédé selon la revendication 1, **caractérisé en ce que** l'étape de mesure de la topographie de la-zone stomale comporte les étapes de balayage de la zone stomale avec un laser.

14. Procédé selon la revendication 1, **caractérisé en ce que** l'étape de mesure de la topographie de la zone stomale comporte l'étape de formation d'un moule de la zone stomale.

15. Procédé selon la revendication 14, **caractérisé en ce que** l'étape de formation d'un moule comporte les étapes de remplissage d'un collier (46) avec un matériau de moule (52), de placement du collier rempli (46) adjacent à la zone stomale et de l'étape consistant à permettre le matériau de moule (52) à se solidifier.

16. Procédé selon la revendication 14, **caractérisé en ce que** l'étape de mesure de la topographie de la zone stomale comporte, en outre, l'étape de balayage de la surface du matériau de moule (52) solidifié avec un laser.

17. Procédé selon la revendication 3, **caractérisé en ce que** l'étape de formation du contour de la pastille adhésive (12, 18) comporte les étapes de sélection d'une pastille avec une dimension qui est supérieure à la dimension sélectionnée et de découpe de la pastille pour qu'elle est un contour en conformité avec la dimension et la forme sélectionnée.

18. Procédé selon la revendication 1, **caractérisé en ce que** l'étape de formation de la pastille adhésive avec le contour de surface sélectionné comporte les étapes de sélection d'un disque convexe avec une ouverture centrale, de fixation du disque convexe sur la pastille et de revêtement de la surface convexe du disque avec un adhésif.

19. Procédé selon la revendication 1, **caractérisé en ce que** l'étape de formation de la pastille adhésive avec le contour de surface sélectionné comporte les étapes de chauffage d'une couche adhésive et de formation de la couche adhésive chauffée dans un moule.

20. Procédé selon la revendication 19, **caractérisé en ce qu'**il comporte, en outre, l'étape d'utilisation d'un matériau de charge comme renfort pour ladite couche adhésive.

21. Procédé selon la revendication 1, **caractérisé en ce que** l'étape de formation de la couche adhésive avec le contour de surface sélectionné comporte la formation d'une pluralité de tranches (71 à 81), chacune avec une partie du contour de surface sélectionné, joignant les tranches (71 à 81) pour former une base et la déposition d'une couche d'adhésif sur ladite base.

22. Procédé selon la revendication 18, **caractérisé en ce qu'**il comporte, en outre, l'étape de sélection d'un insert convexe et d'ajustement par encliquettement de l'insert convexe dans l'ouverture centrale du disque convexe (86).

23. Procédé selon la revendication 18, **caractérisé en ce qu'**il comporte, en outre, les étapes de fixation d'une enceinte annulaire définissant une chambre interne (88) sur la surface de disque convexe (86) avant le revêtement de celui-ci avec un adhésif (64).

24. Procédé selon la revendication 23, **caractérisé en ce qu'**il comporte, en outre, l'étape d'introduction d'un matériau d'altération de contour (96) dans la chambre (88).

25. Procédé selon la revendication 24, **caractérisé en ce qu'**il comprend, en outre, l'étape de manipulation du matériau pour altérer le contour de la pastille.

26. Procédé selon la revendication 24, **caractérisé en ce que** le matériau comprend de l'amidon de maïs.

27. Procédé selon la revendication 24, **caractérisé en ce que** le matériau comprend un fluide.

28. Procédé selon la revendication 24, **caractérisé en ce que** le matériau comprend une résine à durcissement rapide.

29. Procédé selon la revendication 24, **caractérisé en ce que** le matériau comprend une éponge comprimée et comprend, en outre, l'étape d'introduction d'eau dans la chambre pour dilater l'éponge (96).

30. Procédé selon la revendication 4, **caractérisé en ce que** l'étape de formation de la poche comporte les étapes de placement de feuilles de films barrières les unes au-dessus des autres, de soudage de la surface d'une feuille à la surface d'une autre feuille (106) par guidage d'un instrument de soudage suivant un contour déterminé en conformité avec les informations enregistrées pour former la périphérie de poche.

31. Procédé selon la revendication 30, **caractérisé en ce que** l'instrument de soudage comporte un faisceau laser et dans lequel l'étape de guidage comprend la direction du faisceau sur les feuilles.

32. Procédé selon la revendication 30, **caractérisé en ce que** l'instrument de soudage comporte une roue chaude.

33. Procédé selon la revendication 5, **caractérisé en ce qu'**il comporte, en outre, l'étape de fixation de la pastille à la bague de fixation.

34. Procédé selon la revendication 33, **caractérisé en ce qu'**il comporte, en outre, l'étape de fixation d'une première bague de couplage à ladite bague de fixation et de fixation d'une seconde bague de couplage à la pastille.

35. Procédé selon la revendication 1, **caractérisé en ce que** l'étape d'enregistrement des informations de mesure comporte l'étape de marquage d'une carte pour indiquer divers aspects de la topographie de la zone stomale.

36. Procédé selon la revendication 35, **caractérisé en ce que** l'étape de transformation des informations enregistrées sous forme électronique comporte l'étape de conversion des informations sous forme numériques.

37. Procédé selon la revendication 1, **caractérisé en ce que** l'étape d'enregistrement des informations de mesure comporte l'étape de formation de parties simulées de la pastille, d'altération des parties simulées en conformité avec les mesures, et d'assemblage des parties altérées d'une manière indicative de la relation désirée entre les parties.

38. Procédé selon la revendication 37, **caractérisé en ce que** l'étape de transformation des informations enregistrées comporte l'étape de balayage de la surface des parties assemblées et de conversion des informations balayées sous forme numérique.

39. Procédé selon la revendication 1, **caractérisé en ce que** les dispositifs d'ostomie personnalisés comprennent une ouverture de réception de stoma dans la pastille adhésive (42, 18), une bague de fixation (65, 66) définissant la zone dans laquelle ladite ouverture est située, une poche de recueil de déchets (14, 20) avec un orifice d'entrée, et des moyens pour monter ladite poche sur ladite pastille avec ledit orifice d'entrée aligné avec ladite ouverture, dans lesquels la dimension, la forme et l'emplacement de ladite ouverture au sein de ladite zone est sélectionné en conformité avec la dimension, la forme et l'emplacement du stoma de l'utilisateur.

40. Procédé selon la revendication 39, **caractérisé en ce que** ladite pastille (12, 18) a une forme et une dimension sélectionnée en conformité avec la topographie de la zone stomale de l'utilisateur.

41. Procédé selon la revendication 39, **caractérisé en ce que** la poche (14, 20) a un contour sélectionné en conformité avec la topographie de la zone stomale de l'utilisateur.

42. Procédé selon la revendication 39, **caractérisé en ce que** ladite poche comporte une bague de fixation et dans laquelle l'emplacement de ladite bague de fixation sur ladite poche est sélectionnée en conformité avec la topographie de la zone stomale de l'utilisateur.

43. Procédé selon la revendication 39, **caractérisé en ce que** la surface du contour de ladite pastille est sélectionnée en conformité avec la topographie de la zone stomale de l'utilisateur.

44. Procédé selon la revendication 1, **caractérisé en ce que** les dispositifs d'ostomie personnalisés comprennent une ouverture de réception de stoma dans la pastille adhésive (12, 18), un orifice d'entrée dans la poche de recueil, et' un moyen pour fixer ladite pastille (12, 18) et ladite poche (14, 20) avec ladite ouverture alignée avec ledit orifice d'entrée, ledit moyen de fixation comprenant une première bague de fixation soudée à ladite poche, une bride (126) montée, de manière amovible, sur ladite première bague et une seconde bague de fixation soudée à ladite pastille (12, 18), l'une parmi ladite première bague et ladite bride (126) comportant un canal annulaire (132), ladite seconde bague comprenant une saillie annulaire adaptée pour être reçue, de manière amovible, dans ledit canal (132).

45. Procédé selon la revendication 44, **caractérisé en ce que** ladite première bague comporte une paroi extérieure, le dispositif comporte, en outre, des sailles (138) sur ladite paroi espacée pour recevoir ladite bride (126) entre celles-ci.

46. Procédé selon la revendication 45, **caractérisé en ce que** ladite bride est formée d'un matériau suffisamment flexible pour passer sur les saillies (138) dans une direction.

47. Procédé selon la revendication 44, **caractérisé en ce que** le dispositif comporte, en outre, des moyens pour monter, de manière amovible, ladite bride (126) sur ladite première bague.

48. Procédé selon la revendication 47, **caractérisé en ce que** ledit moyen de montage amovible comporte un évidemment dans ladite première bague et une saillie sur ladite bride (126) adaptée pour être reçue dans ledit évidemment.

49. Procédé selon la revendication 1, **caractérisé en ce que** le dispositif d'ostomie personnalisé comporte un moyen pour monter ladite poche (14, 20) sur ladite pastille (12, 18) et une rondelle (156) de montage de coulée fixée à une paroi de ladite poche (14, 20) sur une ouverture dans ladite paroi, ladite rondelle (156) comportant une ouverture centrale (160) alignée avec ladite ouverture de poche, une seconde ouverture (162) décalée de ladite ouverture centrale, un disque amovible dans ladite seconde ouverture (162), et une coulée comportant une base avec un montant central (158) adapté pour être reçue dans ladite ouverture centrale (160) dans ladite rondelle (156).

50. Procédé selon la revendication 49, **caractérisé en ce que** ledit montant (158) est creux et agit pour raccorder ladite coulée à l'intérieur de ladite poche (14, 20).

51. Procédé selon la revendication 49, **caractérisé en ce que** ledit montant (158) est solide et agit comme un axe lorsque la base est tournée par rapport à ladite rondelle (156).

52. Procédé selon la revendication 51, **caractérisé en ce que** ladite coulée comporte une conduite et **en ce que** ladite conduite se termine dans ladite base de manière à s'aligner avec ladite seconde ouverture (162) lorsque ladite base est dans une position de rotation sélectionnée par rapport à ladite rondelle (156).

53. Procédé selon la revendication 1, **caractérisé en ce que** le dispositif comporte une pince de queue comportant des première et seconde parties d'interaction (166, 168, 170, 172), chacune desdites parties étant entaillées à des intervalles réguliers pour faciliter l'équilibrage de chacun à essentiellement la même longueur.
